# FASCICULE DE BREVET EUROPEEN

(11) **EP 0 870 046 B1**
(45) Date de publication et mention de la délivrance du brevet: **07.01.2009**
(21) Numéro de dépôt: 96939150.7
(22) Date de dépôt: 19.11.1996
(51) Int. Cl.: C12N 15/86, C07K 14/165, A61K 39/245, A61K 39/215

(54) **VACCIN VIVANT RECOMBINANT A BASE D'HERPESVIRUS FELIN DE TYPE 1, NOTAMMENT CONTRE LA PERITONITE INFECTIEUSE FELINE**
REKOMBINANTER LEBENDIMPFSTOFF, DER KATZENHERPESVIRUS TYP I ENTHÄLT, INSBESONDERE FÜR DIE BEHANDLUNG VON INFEKTIÖSER BAUCHFELLENTZÜNDUNG BEI KATZEN
RECOMBINANT LIVE VACCINE CONTAINING FELINE HERPES VIRUS TYPE 1, PARTICULARLY FOR TREATING FELINE INFECTIOUS PERITONITIS

(30) Priorité: 30.11.1995 FR 9514450
(43) Date de publication de la demande: 14.10.1998
(73) Titulaire: Mérial, 69002 Lyon (FR)
(72) Inventeur: AUDONNET, Jean-Christophe, Francis, F-69006 Lyon (FR); BAUDU, Philippe, Guy, Nicolas, F-69005 Lyon (FR); RIVIERE, Michel, Albert, Emile, F-69130 Ecully (FR)
(74) Mandataire: Nargolwalla, Cyra
(86) Numéro de dépôt international: PCT/FR1996/001830
(87) Numéro de publication internationale: WO 1997/020059

(56) Documents cités:
- EP-A- 0 447 303
- EP-A- 0 576 092
- WO-A-91/01332
- WO-A-95/07987
- WO-A-95/30019
- J. GEN. VIROL., vol. 75, 1994, pages 3107-3116, XP002013244 M. WILLEMSE ET AL.: "The gene downstream of the gC homolog in feline herpes virus type 1 is involved in the expression of virulence"
- J. GEN. VIROL., vol. 73, 1992, pages 1811-1818, XP000288657 R. WARDLEY ET AL.: "The use of feline herpesvirus and baculovirus as vaccine vectors for the gag and env genes of feline leukemia virus" cité dans la demande

## Description

La présente invention a trait à des vaccins, de préférence pour chats, produits à partir d'herpèsvirus félins recombinants, et aux méthodes d'obtention et de préparation de ces virus recombinants. En particulier, la présente invention a plus particulièrement trait aux recombinants herpèsvirus félins comprenant une cassette d'expression pour un (ou des) gène(s) étranger(s).

La rhinotrachéite infectieuse féline est causée par l'herpèsvirus félin de type 1 (en anglais Feline HerpesVirus Type 1 ou FHV-1). L'herpèsvirus félin (FHV-1) est classé dans la famille des *Alphaherpesvirinae.* La rhinotrachéite infectieuse féline est une maladie très répandue chez les chats et, en pratique, tous les chats médicalisés sont vaccinés contre cette affection virale. Il existe actuellement plusieurs vaccins pour prévenir la rhinotrachéite infectieuse. Ces vaccins sont soit de type vivant atténué, soit de type inactivé (virus entier ou sous-unités purifiées). L'atténuation des vaccins vivants actuellement utilisés a été obtenue après passages répétés sur cellules, et la cause de leur atténuation n'est pas connue. De plus, ces vaccins présentent en général une virulence résiduelle et sont pour cette raison administrés par voie parentérale (sous-cutanée ou intramusculaire) plutôt que par voie intranasale (qui serait cependant la voie de choix compte tenu de la réplication locale de ce virus). Les vaccins inactivés présentent une bonne innocuité, mais leur faible immunogénicité nécessite de multiples injections pour induire une protection satisfaisante.

Par ailleurs, les chats domestiques sont exposés à de nombreuses autres maladies, et la mise au point d'un vecteur vaccinal pouvant exprimer différents antigènes d'agents pathogènes félins permettrait de simplifier et d'améliorer l'efficacité des programmes de vaccination.

Enfin, parmi les maladies affectant le chat domestique, certaines résistent toujours aux approches vaccinales classiques. Le cas le plus connu est celui de la péritonite infectieuse féline causée par un coronavirus (virus de la Péritonite Infectieuse Féline (PIF) ou, en anglais, "FIPV" (Feline Infectious Peritonitis Virus)).

Un vecteur FHV-1, dont l'atténuation serait telle qu'il puisse être administré par voie oronasale chez le chat sans entraîner de pathologie locale et/ou générale, et qui permettrait l'induction d'une réponse immunitaire protectrice à la fois contre la rhinotrachéite infectieuse féline et contre d'autres agents pathogènes félins, constituerait un progrès significatif en matière de vaccination de la population féline domestique.

On a déjà proposé un certain nombre de gènes de FHV comme sites d'insertion :
La demande de brevet EP-A-0 447 303 a proposé l'insertion dans le site RR2 des alphaherpèsvirus, y compris FHV. La demande donne les moyens d'effectuer l'insertion dans le site RR2 de l'herpèsvirus de la dinde (virus HVT). La demande de brevet WO-A-90 01547 propose des vecteurs FHV TK- pour l'expression de gènes hétérologues.
De même, la demande de brevet WO-A-93 09238 propose un vaccin contre la leucémie féline formée d'un vecteur FHV dans lequel un gène de FeLV a été inséré dans le gène TK du virus FHV. Voir aussi dans le même sens les articles de R.C. WARDLEY et al. dans J. Gen. Virol. 1992. 73. 1811-1818 et de G.E. COLE et al. dans J. Virol. 1990. 64. 4930-4938.
La demande de brevet WO-A-94 03621 propose l'insertion dans les gènes gl, gE, US9, US10 et US11.
La demande de brevet WO-A-95 00172 propose d'insérer un ADN servant de marqueur dans la région du génome comprenant les gènes gl et gE.
La demande de brevet EP-A-0 576 092 propose le cadre ouvert de lecture ("ORF") situé entre le gène gC et le gène homologue du gène HSV-1 UL46 comme site préférentiel pour l'insertion sur le génome FHV. Voir aussi M.J. WILLEMSE et al. dans J. Gen. Virol. 1994. 75. 3107-3116.

Divers promoteurs, y compris ceux généralement disponibles dans le commerce, ont été utilisés dans les différentes constructions décrites dans l'art antérieur, parmi lesquels le promoteur HCMV IE (CMV immediate early humain), la séquence promotrice de la région LTR du virus RSV (Rous Sarcoma Virus), et le promoteur précoce du virus SV40.

La présente invention a pour objectif de fournir un vaccin FHV vivant, atténué mais ayant conservé une bonne capacité de réplication *in vivo*, en vue d'immuniser les chats contre la rhinotrachéite infectieuse.

Un autre objectif de l'invention est de fournir un vaccin vivant recombinant à base de FHV qui soit efficace contre les autres agents pathogènes félins. En particulier, en raison des nombreux échecs observés dans la vaccination contre la péritonite infectieuse féline avec des vaccins inactivés ou vivants atténués, principalement en raison du phénomène de "facilitation" (exacerbation de la maladie), le besoin d'un vaccin réellement efficace contre la PIF existe toujours. Un tel vaccin à base d'un vecteur FHV-1 recombinant qui serait réellement efficace contre la péritonite infectieuse féline, maladie pour laquelle personne n'a encore commercialisé de vaccin satisfaisant, pourrait en outre ouvrir la voie à des vaccins très efficaces contre d'autres maladies du chat, comme, par exemple et entre autres, la leucémie féline, le syndrome d'immunodéficience féline dû à FIV, ou encore la panleucopénie féline.

Un autre objectif encore de l'invention est de permettre une vaccination efficace des chats en utilisant la voie oronasale.

La demanderesse a caractérisé une nouvelle partie du génome de FHV, dans laquelle elle a caractérisé de nouvelles régions du génome du virus FHV, régions dénommées ci-après (voir exemple 3) FHV ORF2 et FHV ORF5, qui se sont avérées utilisables pour l'insertion de gènes étrangers dans des conditions permettant de remplir les objectifs exposés ci-dessus.

La présente invention a donc pour objet un vaccin vivant recombinant utilisant comme vecteur un herpèsvirus félin comprenant, et exprimant, au moins une séquence nucléotidique codant pour un polypeptide, cette séquence étant insérée dans le site ORF5 et/ou le site ORF2.

De préférence, la séquence insérée code pour un polypeptide antigénique et, préférentiellement, pour un polypeptide antigénique d'un agent pathogène félin. On peut également insérer des séquences codant pour des protéines immunomodulatrices telles que des cytokines. Selon une modalité avantageuse, on peut associer une séquence codant pour une cytokine, ou analogue, et une séquence codant pour un antigène. Si besoin est, plusieurs séquences de cytokines peuvent être associées entre elles, éventuellement en combinaison avec une ou des séquences codant pour des antigènes.

L'insertion dans les deux sites nouvellement caractérisés est réalisée par insertion simple (sans délétion) ou après délétion partielle ou totale des ORFs utilisées comme sites d'insertion.

Selon une modalité particulièrement préférée de l'invention, on effectue des insertions et/ou des délétions dans les deux sites décrits. Cette modalité est particulièrement adaptée à l'utilisation de souches sauvages virulentes du virus FHV-1 pour l'obtention de recombinants. On peut donc insérer au moins une séquence nucléotidique dans chacun des sites, ou encore n'insérer que dans un site, de préférence ORF5, et déléter tout ou partie de l'autre site.

Selon une autre modalité, qui s'applique plus aux souches vaccinales atténuées, sans y être limitée, l'insertion est effectuée dans un seul des deux sites, de préférence le site ORF5.

Les herpèsvirus félins selon l'invention sont de préférence les virus FHV de type 1.

On peut utiliser en particulier la souche FHV-1 CO dont la séquence de la région génomique (ORF1 à ORF8) est indiquée dans la liste de séquences sous la référence SEO ID N° 1 (voir aussi tableau 1 exemple 3). Le site ORF2 est situé entre les nucléotides 1655 et 2596. Le site ORF5 est situé entre les nucléotides 5869 et 7113.

Pour l'expression des gènes étrangers insérés sur le génome de FHV-1 selon la présente invention, on utilisera de préférence un promoteur eucaryote fort, tel que, préférentiellement, un promoteur CMV immediate early (IE). Par promoteur CMV IE, on entend notamment un fragment tel que donné dans les exemples ainsi que ses sous-fragments conservant la même activité promotrice. Le promoteur CMV IE peut être le promoteur humain (HCMV IE) ou le promoteur murin (MCMV IE), ou encore un promoteur CMV IE d'une autre origine, par exemple du rat, du cobaye, ou du porc.

On pourra insérer au moins deux séquences nucléotidiques dans l'un des sites ORF2 ou ORF5 sous le contrôle de promoteurs différents. Il pourra notamment s'agir de promoteurs CMV IE d'origines différentes.

Selon un développement avantageux de l'invention, on associe au promoteur CMV IE un autre promoteur selon une disposition tête-bêche, telle que les deux promoteurs ont leurs extrémités 5' adjacentes, si bien que les transcriptions initiées à partir de ces promoteurs se font en sens opposés, ce qui permet d'insérer, dans la région d'insertion, deux séquences nucléotidiques, l'une sous la dépendance du promoteur CMV IE, l'autre sous celle du promoteur associé. Cette construction est remarquable par le fait que la présence du promoteur CMV IE, et notamment de sa partie activatrice (enhancer), peut activer la transcription induite par le promoteur associé. Comme promoteur associé, on peut citer, par exemple, un promoteur CMV d'espèce différente du premier promoteur. On peut également envisager d'autres promoteurs tels que le promoteur RNA1.8 du virus de la maladie de Marek (MDV) (G. Bradley et al. J. Virol. 1989. 63.2534-2542).

La séquence nucléotidique insérée dans le vecteur FHV pour être exprimée peut être toute séquence codant pour un polypeptide antigénique d'un agent pathogène félin capable, une fois exprimée dans les conditions favorables procurées par l'invention, d'assurer une immunisation conduisant à une protection efficace de l'animal vacciné contre l'agent pathogène. On pourra donc insérer, dans les conditions décrites par la présente l'invention, les séquences nucléotidiques codant pour les antigènes d'intérêt pour une maladie donnée.

Le cas typique de l'invention est l'insertion d'au moins une séquence nucléotidique codant convenablement pour un polypeptide du virus de la péritonite infectieuse féline (PIF ou FIPV) et, de préférence, le polypeptide FIPV M ou le polypeptide FIPV S modifié. On obtient ainsi un vaccin vivant recombinant assurant, en plus d'une protection contre la rhinotrachéite infectieuse féline, une protection contre la péritonite infectieuse féline. Si on le souhaite, on peut aussi insérer, en sus ou à la place, une séquence codant pour un autre antigène du virus PIF, tel que la protéine N, la protéine 7b, et/ou encore les polypeptides codés par le gène polymérase (polB) du virus de la PIF.

D'autres cas préférés de l'invention sont l'insertion de séquences nucléotidiques codant pour des antigènes ou des fragments d'antigènes du virus de la leucémie féline (FeLV), en particulier gènes env, gag et pol (Osterhaus A. et al. J. Immunol., 1985. 135. 591-596; Lutz H. Vet. Microbiol. 1990. 23. 131-146; Clark N. et al. JAVMA, 1991. 199. 1433-1443; Thomsen D. et al. J. Gen. Virol., 1992. 73. 1819-1824), du virus de l'immunodéficience féline (FIV) (Jarrett O. et al. AIDS, 1990, 4 (suppl. 1): S163-S165; Miyazawa T. et al. Arch. Virol; 1994, 134, 221-234; de Rhonde A. et al. Virology, 1994. 198. 257-264), en particulier gènes env, gag et pol, du virus de la panieucopénie féline (FPV) (Carlson J. et al. J. Virol. 1985. 55. 574-582; Martyn J. et al. J. Gen. Virol. 1990. 71, 2747-2753), en particulier le gène de capside VP2, du calicivirus félin (FCV) (Neill J. et al. J. Virol. 1991. 65. 5440-5447; Carter M. et al. Virology. 1992. 190. 443-448), en particulier le gène de capside.

Un cas typique de l'invention est un vaccin comprenant une séquence nucléotidique codant pour un antigène du virus de la PIF sous le contrôle de CMV IE et une séquence nucléotidique codant pour un antigène d'une autre maladie virale féline, notamment celles citées plus haut, sous le contrôle de l'autre promoteur.

La présente invention a aussi pour objet une formule de vaccin multivalent, comprenant, en mélange ou à mélanger, au moins deux vaccins vivants recombinants tels que définis plus haut, ces vaccins comprenant des séquences insérées différentes, notamment de pathogènes différents.

La présente invention a aussi pour objet les virus FHV modifiés dans l'un ou les deux sites ORF2 et ORF5 comme indiqué ci-dessus.

Elle a aussi pour objet une méthode de vaccination, en particulier des chats, dans laquelle on administre par toute voie parentérale ou locale, mais de préférence par voie oronasale, une quantité efficace d'un vaccin tel que défini plus haut. La dose vaccinale sera comprise entre 10² DICC50 et 10⁷ DICC50. Tel que défini, le vaccin est efficace en général après une seule administration par voie oronasale. Cependant, des administrations répétées peuvent être nécessaires.

La présente invention a aussi pour objet les fragments d'ADN comprenant tout ou partie de la séquence définie par les positions 1 à 8193 sur SEQ ID N° 1, notamment tout ou partie des sites ORF2 et ORF5 définis et/ou des séquences flanquantes localisées en amont et en aval de ces sites, fragments qui seront utiles comme bras flanquants pour les techniques de recombinaison homologue avec le génome du virus FHV parental. Bien entendu, l'invention concerne aussi les variantes de ces fragments qui correspondent aux séquences équivalentes des autres souches de FHV. Le spécialiste a toute latitude pour choisir les régions servant de bras flanquants en liaison avec le type d'insertion (avec ou sans délétion) ou de délétion (partielle ou totale) choisi. D'une manière générale, les bras flanquants pourront ainsi avoir de 100 à 800 paires de bases.

L'invention va être maintenant décrite plus en détail à l'aide d'exemples de réalisation non limitatifs, pris en référence au dessin, dans lequel :
- **Figure 1 :**: Séquence de la région FHV-1 (10803 paires de bases) et traduction des différents cadres ouverts de lecture (ORF) présents sur cette séquence (ORF1 à ORF8).
- **Figure 2 :**: Construction du plasmide pPB107 (plasmide donneur pour l'insertion de cassettes d'expression dans le site FHV-1 ORF2)
- **Figure 3 :**: Construction du plasmide pPB110 (plasmide donneur pour l'insertion de cassettes d'expression dans le site FHV-1 ORF5)
- **Figure 4 :**: Construction de la cassette d'expression pour le gène FIPV M (plasmide pPB105)
- **Figure 5 :**: Construction de la cassette d'expression pour le gène FIPV S (plasmide pPB055)
- **Figure 6 :**: Mutagénèse du site A1 du gène FIPV S (plasmide pJCA084)
- **Figure 7 :**: Mutagénèse du site A2 du gène FIPV S (plasmide pJCA085)
- **Figure 8 :**: Mutagénèse des sites A1 + A2 du gène FIPV S (= FIPV S*) (plasmide pJCA087)
- **Figure 9 :**: Construction de la cassette d'expression pour le gène FIPV S * modifié (mutations dans les sites A1 et A2) (plasmide pPB056)
- **Figure 10 :**: Construction de la cassette d'expression pour le gène FIPV N (plasmide pJCA091)
- **Figure 11 :**: Construction du plasmide donneur pour l'insertion de la cassette d'expression du gène FIPV M dans le site FHV-1 ORF2 (pPB111)
- **Figure 12 :**: Construction du plasmide donneur pour l'insertion de la cassette d'expression du gène FIPV S * dans le site FHV-1 ORF2 (pPB112)
- **Figure 13 :**: Construction du plasmide donneur pour l'insertion de la cassette d'expression du gène FIPV N dans le site FHV-1 ORF2 (pPB113)
- **Figure 14 :**: Construction du plasmide donneur pour l'insertion de la cassette d'expression du gène FIPV M dans le site FHV-1 ORF5 (pPB114)
- **Figure 15 :**: Construction du plasmide donneur pour l'insertion de la cassette d'expression du gène FIPV S * dans le site FHV-1 ORF5 (pPB115)
- **Figure 16 :**: Construction du plasmide donneur pour l'insertion de la cassette d'expression du gène FIPV N dans le site FHV-1 ORF5 (pPB116)

### Liste des séquences SEQ ID pour les constructions dans les sites ORF2 et ORF5

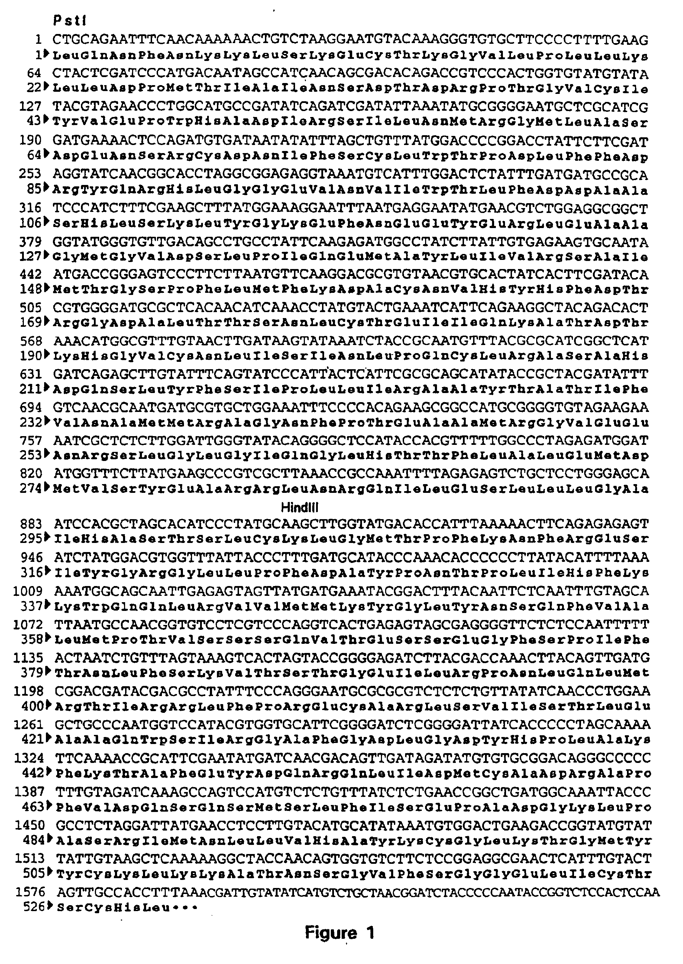

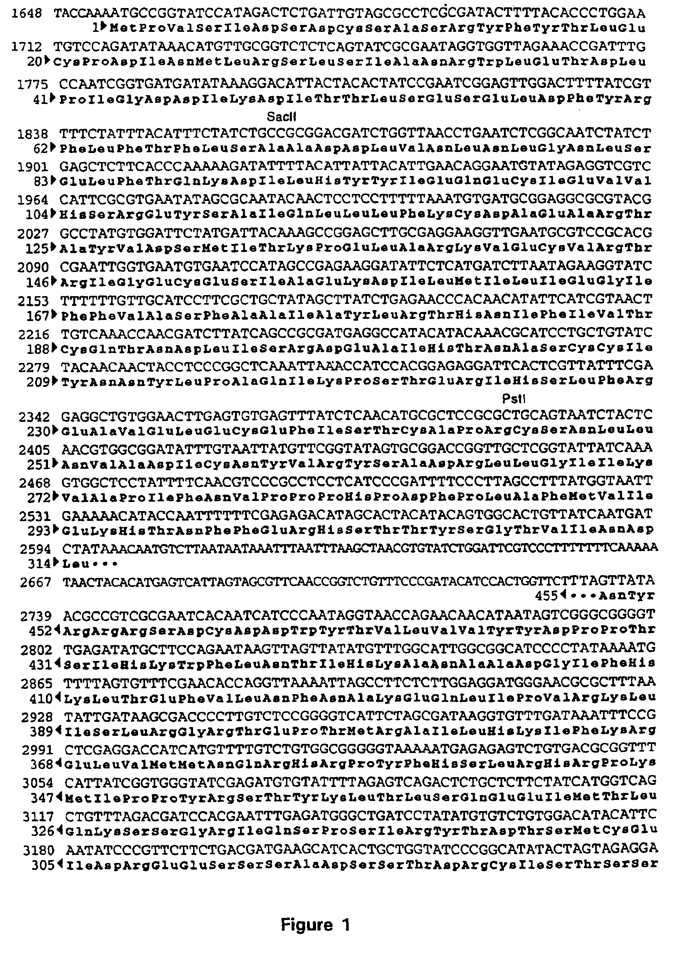

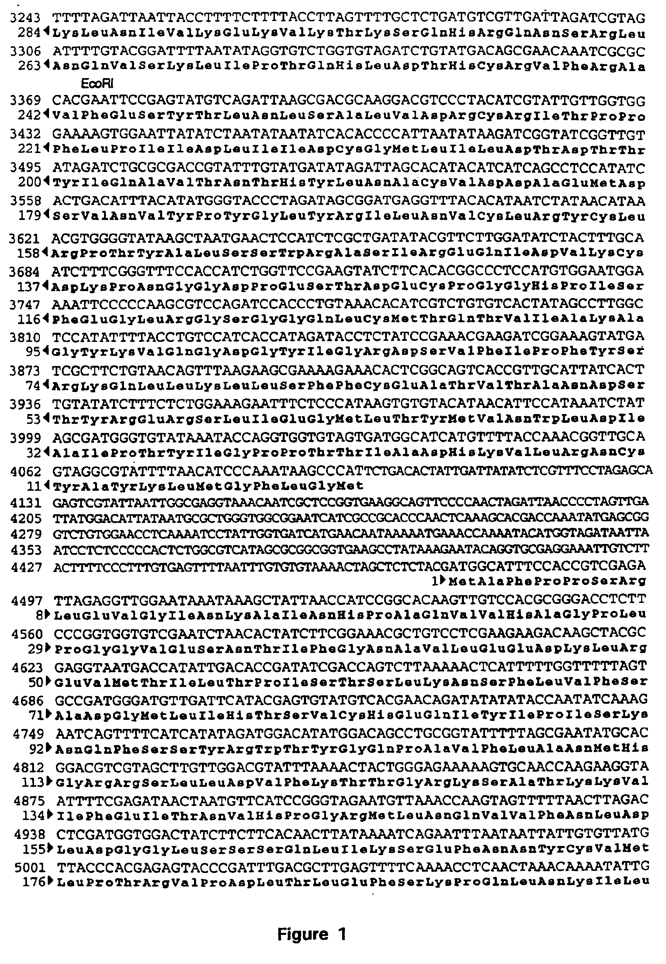

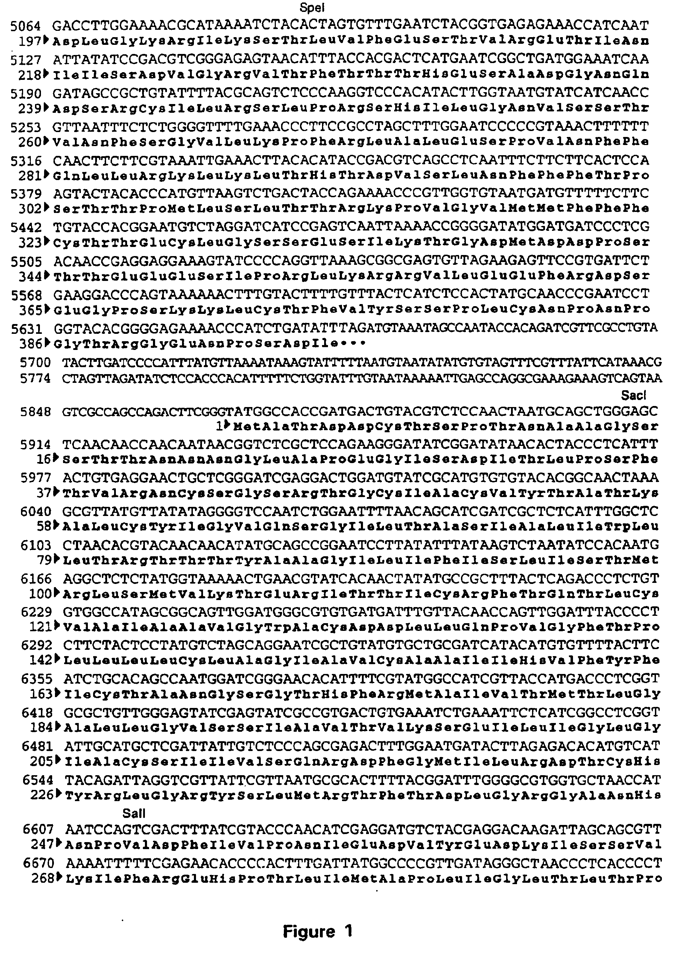

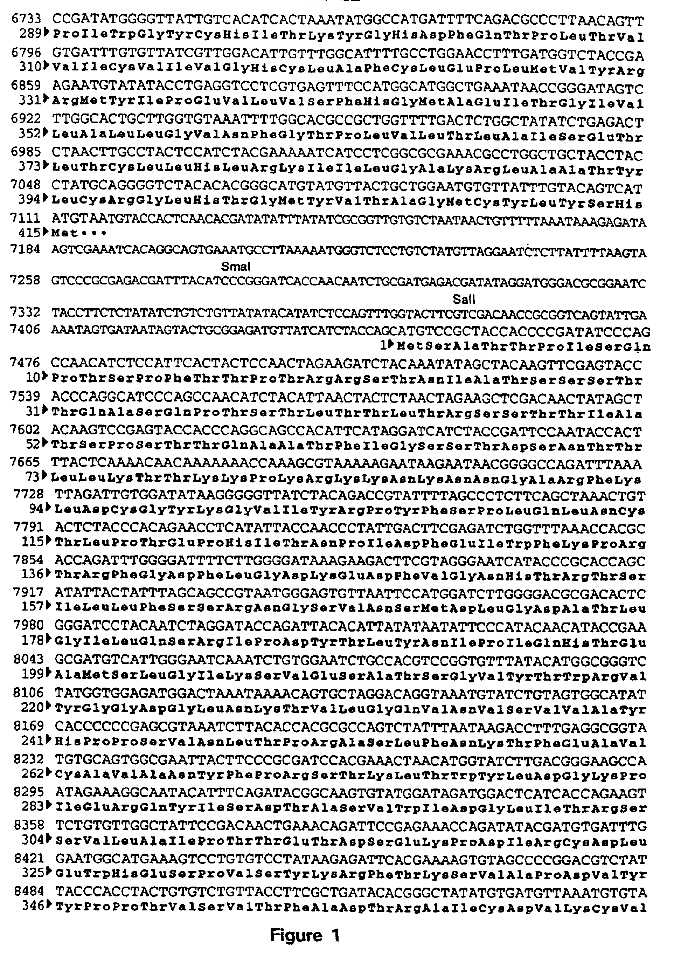

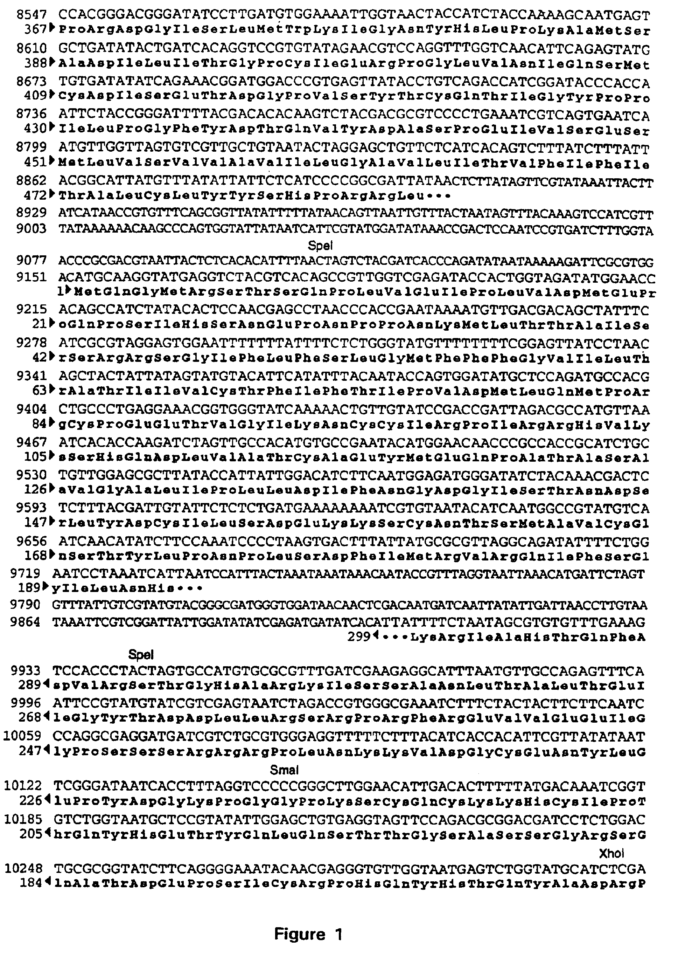

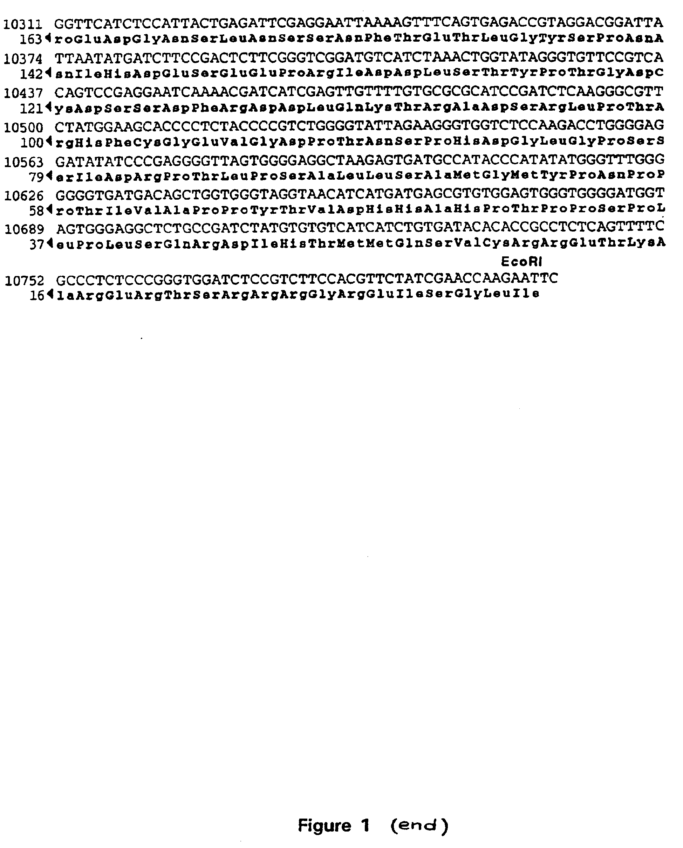

- **SEQ ID N° 1**: Séquence complète de la région FHV-1 ORF1 --> ORF8 représentée à la figure 1
- **SEQ ID N° 2**: Séquence acides aminés partielle ORF FHV-1 ORF1 de la figure 1
- **SEQ ID N° 3**: Séquence acides aminés ORF FHV-1 ORF2 de la figure 1
- **SEQ ID N° 4**: Séquence acides aminés ORF FHV-1 ORF3 de la figure 1
- **SEQ ID N° 5**: Séquence acides aminés ORF FHV-1 ORF4 de la figure 1
- **SEQ ID N° 6**: Séquence acides aminés ORF FHV-1 ORF5 de la figure 1
- **SEQ ID N° 7**: Séquence acides aminés ORF FHV-1 ORF6 de la figure 1
- **SEQ ID N° 8**: Séquence acides aminés ORF FHV-1 ORF7 de la figure 1
- **SEQ ID N° 9**: Séquence acides aminés partielle ORF FHV-1 ORF8 de la figure 1
- **SEQ ID N° 10**: Oligonucléotide JCA054
- **SEQ ID N° 11**: Oligonucléotide JCA055
- **SEQ ID N° 12**: Oligonucléotide PB080
- **SEQ ID N° 13**: Oligonucléotide PB081
- **SEQ ID N° 14**: Oligonucléotide PB082
- **SEQ ID N° 15**: Oligonucléotide PB083
- **SEQ ID N° 16**: Oligonucléotide PB084
- **SEQ ID N° 17**: Oligonucléotide PB085
- **SEQ ID N° 18**: Oligonucléotide JCA056
- **SEQ ID N° 19**: Oligonucléotide JCA057
- **SEQ ID N° 20**: Oligonucléotide PB088
- **SEQ ID N° 21**: Oligonucléotide PB089
- **SEQ ID N° 22**: Oligonucléotide JCA058
- **SEQ ID N° 23**: Oligonucléotide JCA059
- **SEQ ID N° 24**: Oligonucléotide JCA060
- **SEQ ID N° 25**: Oligonucléotide JCA061
- **SEQ ID N° 26**: Oligonucléotide JCA062
- **SEQ ID N° 27**: Oligonucléotide JCA063
- **SEQ ID N° 28**: Oligonucléotide JCA064
- **SEQ ID N° 29**: Oligonucléotide JCA065
- **SEQ ID N° 30**: Oligonucléotide JCA066
- **SEQ ID N° 31**: Oligonucléotide JCA067
- **SEQ ID N° 32**: Oligonucléotide JCA068
- **SEQ ID N° 33**: Oligonucléotide JCA069

### 2. EXEMPLES

Toutes les constructions de plasmides ont été réalisées en utilisant les techniques standards de biologie moléculaire décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

Le virus utilisé comme virus parental est la souche CO de l'herpèsvirus félin de type 1 (FHV-1). Ce virus a été isolé à partir de cellules rénales d'un chaton nouveau-né dont la mère était atteinte de rhinotrahéite infectieuse (C. Benoit Jeannin, Thèse de Doctorat de 3ème cycle, Université de Lyon, 1983). Les conditions de culture de ce virus ont déjà été décrites (Fargeaud D. *et al*. Arch. Virol. 1984. **80.** 69-82). Brièvement, des cellules CRFK ("Crandell Rees Feline Kidney cells"), cultivées en milieu minimum essentiel de Eagle (milieu "MEM) sont inoculées avec la souche FHV-1 CO en utilisant une multiplicité d'infection de 1. Les cellules infectées sont alors incubées à 37°C pendant environ 36 heures, juqu'à apparition d'un effect cytopathique complet.

### Exemple 1: Extraction de l'ADN de l'herpèsvirus félin de type 1:

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile.

### Exemple 2: Isolement de l'ARN génomique de la souche FIPV 79-1146 et clonage de l'ADN complémentaire

La souche FIPV 79-1146 a été cultivée sur cellules CRFK en milieu DMEM (Gibco). L'ARN viral génomique a été isolé en utilisant la technique d'extraction thiocyanate de guanidium/phénol-chloroforme (Chomczynski P. et Sacchi N., Anal. Biochem. 1987. 162. 156-159). Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (respectivement M, S et N). La réaction de transcription inverse (RT) et l'amplification en chaîne par polymérase (PCR) ont été effectuées selon les techniques standards (Sambrook J. *et al*. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction.

### Exemple 3: Clonage et caractérisation de la région FHV-1 ORF1 - ORF7 (fragments Ecorl D et EcoRI F)

L'ADN génomique purifié de la souche CO du virus FHV-1 a été digéré par *EcoRI* et les fragments D (environ 9200 pb) et F (7600 pb) ont été clonés dans le vecteur pBlueScript SKIl + pour donner respectivement les plasmides pFHVEcoRID et pFHVEcoRIF. Le plasmide pFHVEcoRID a été digéré par *Eco*RI et *Pst*I et le fragment EcoRI-PstI de 979 pb a été isolé et ligaturé avec le vecteur pBS-SKII +, préalablement digéré par *Eco*RI et *Pst*I pour donner le plasmide pPB050. Le plasmide pFHVEcoRID a été digéré par *Pst*I et le fragments PstI-PstI de 2388 pb a été isolé et ligaturé avec le vecteur pBS-SKII+, préalablement digéré par *Pst*I pour donner le plasmide pPB051. Les inserts contenus dans les plasmides pFHVEcoRIF, pPB050 et pPB051 ont été entièrement séquencés sur les deux brins pour donner la séquence de la figure 1 (SEQ ID N° 1).

Plusieurs cadres ouverts de lecture de taille supérieure à 65 acides aminés ont été identifiés sur cette séquence (figure 1).

Le premier cadre de lecture (ORF1) (positions 1 - 1587) est incomplet et code pour une protéine tronquée de 529 acides aminés (SEQ ID N° 2).

Le deuxième cadre de lecture (ORF2) (positions 1655 - 2596) code pour un polypeptide de 314 acides aminés (SEQ ID N° 3).

Le troisième cadre de lecture (ORF3) (positions 2733 - 4094) est situé sur le brin complémentaire et code pour un polypeptide de 454 acides aminés (SEQ ID N° 4).

Le quatrième cadre de lecture (ORF4) (positions 4476 - 5660) code pour un polypeptide de 395 acides aminés (SEQ ID N° 5).

Le cinquième cadre de lecture (ORF5) (positions 5869 - 7113) code pour un polypeptide de 415 acides aminés (SEQ ID N°6).

Le sixième cadre de lecture (ORF6) (positions 7449 - 8900) code pour un polypeptide de 484 acides aminés (SEQ ID N°7).

Le septième cadre de lecture identifié sur la séquence de la figure 1 (ORF7) (positions 9153 - 9731) code pour une protéine de 193 acides aminés (SEQ ID N° 8).

Le huitième et dernier cadre de lecture identifié sur la séquence de la figure 1 (ORF8) (positions 9908 - 10803) est incomplet. Il est situé sur le brin complémentaire et code pour une protéine tronquée de 298 acides aminés (SEQ ID N° 9).

Les différents cadres ouverts de lecture sont rassemblés dans le tableau ci-dessous : (Tableau 1)

| Cadre ouvert de lecture | Début - Fin (positions sur figure 1) | Traille en acides aminés |
|---|---|---|
| ORF 1 | 1 - 1587 | 529 aa |
| ORF 2 | 1655 - 2596 | 314 aa |
| ORF 3 | 4094 - 2733 | 454 aa |
| ORF 4 | 4476 - 5660 | 395 aa |
| ORF 5 | 5869 - 7113 | 415 aa |
| ORF 6 | 7449 - 8900 | 484 aa |
| ORF 7 | 9153 - 9731 | 193 aa |
| ORF 8 | 10803 - 9908 | 298 aa |

On pense que le cadre ouvert de lecture FHV ORF2 nouvellement caractérisé est l'homologue du gène HSV-1 UL40 (RR2).

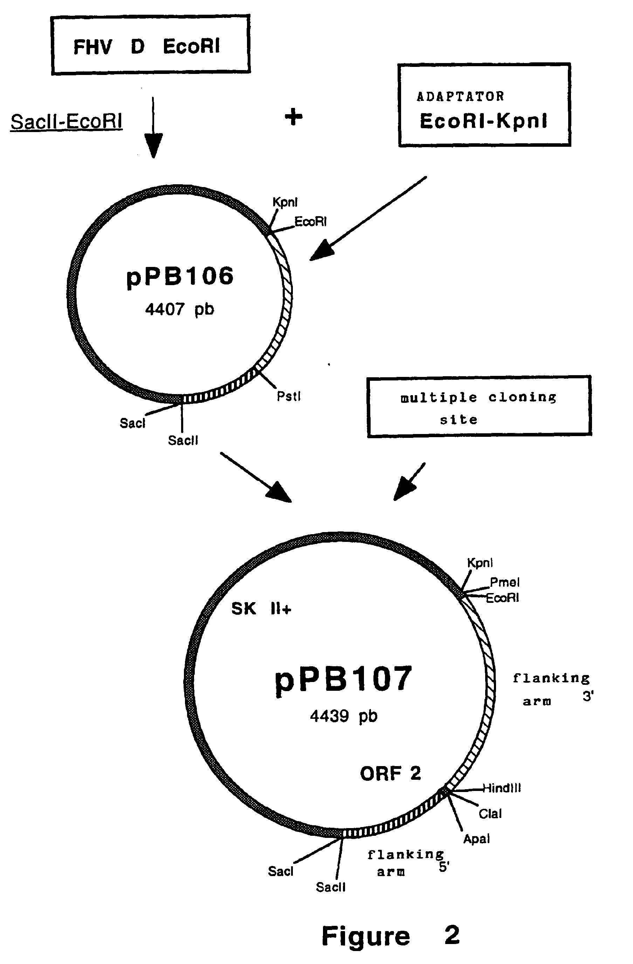

### Exemple 4: Construction du plasmide donneur pour le site FHV-1 ORF2 (pPB107) (figure n° 2)

Le plasmide pFHVEcoRID a été digéré par *Eco*RI et Sacll pour isoler le fragment EcoRI-SacII de 1509 pb. Un adaptateur KpnI-EcoRI contenant le site PmeI a été obtenu par hybridation des 2 oligonucléotides synthétiques suivants :
JCA054 (24 mer) (SEQ ID N° 10):
   5' CTTGCCGGGGTTTAAACCGGTTCG 3'
et JCA055 (32 mer) (SEQ ID N° 11):
   5' AATTCGAACCGGTTTAAACCCCGGCAAGGTAC 3'

Le fragment EcoRI-SacII et l'oligonucléotide double brin ont été ligaturés dans le vecteur pBS-SKII +, préalablement digéré par *Kpn*I et *Sac*II, pour donner le plasmide pPB106 (4407 pb). Un oligonucléotide synthétique double brin comportant les sites de clonage HindIII, ClaI et ApaI a été obtenu par hybridation des 2 oligonucléotides suivants:
PB080 (32 mer) (SEQ ID N° 12):
   5' TGCAAAGCTTATCGATCCCGGGGCCCGGTGCA 3'
et PB081 (32 mer) (SEQ ID N° 13):
   5' CCGGGCCCCGGGATCGATAAGCTTTGCATGCA 3'
L'oligonucléotide ainsi obtenu a été ligaturé avec le plasmide pPB106, prélablement digéré par *Pst*I (site unique sur pPB106) et traité avec la phosphatase alcaline, pour donner le plasmide pPB107 (4439 pb) (figure n° 2). Ce plasmide contient les bras flanquants 5' (SacII-PstI 530 pb) et 3' (PstI-EcoRI 979 pb) du site FHV-1 ORF2 ainsi qu'un site multiple de clonage permettant l'insertion d'une cassette d'expression.

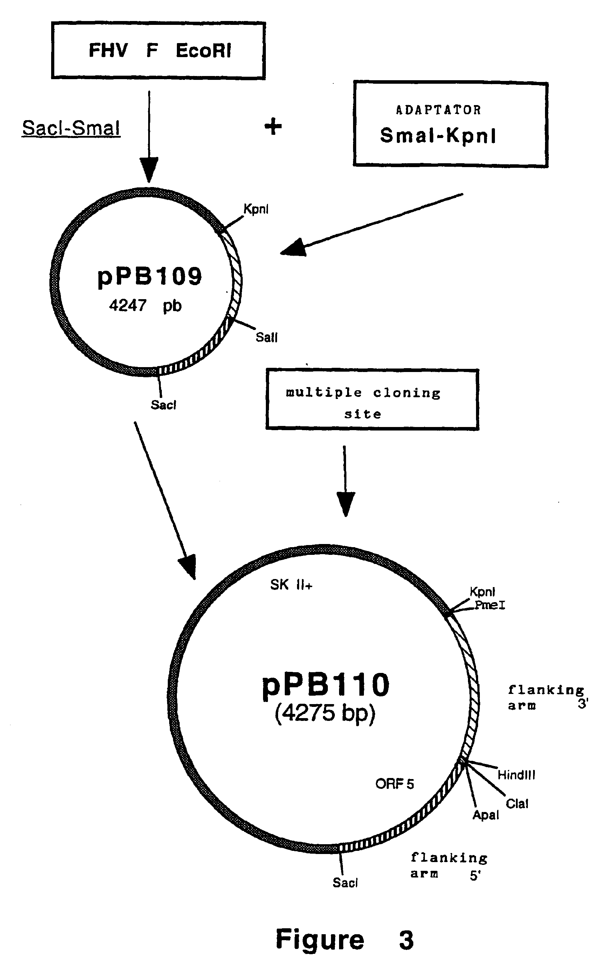

### Exemple 5: Construction du plasmide donneur pour le site FHV-1 ORF5 (pPB110) (figure n° 3)

Le plasmide pFHVEcoRIF (voir exemple 3) a été digéré par *Sac*I et *Sma*I pour isoler le fragment SacI-SmaI de 1367 pb. Un adaptateur SalI-KpnI contenant le site PmeI a été obtenu par hybridation des 2 oligonucléotides suivants:
PB082 (21 mer) (SEQ ID N° 14):
   5' GGGGGCCGTTTAAACCGGTAC 3'
PB083 (17 mer) (SEQ ID N° 15):
   5' CGGTTTAAACGGCCCCC 3'

L'oligonucléotide double brin ainsi obtenu et le fragment SacI-SmaI 1367 pb ont été ligaturés avec le vecteur pBS-SKII +, préalablement digéré par *Kpn*I et *Sac*I pour donner le plasmide pPB109 (4247 pb). Un site multiple de clonage a été obtenu par hybridation des 2 oligonucléotides synthétiques suivants:
PB084 (28 mer) (SEQ ID N° 16):
   5' TCGAGAAAGCTTATCGATCCCGGGCCCG 3'
PB085 (28 mer) (SEQ ID N° 17):
   5' TCGACGGGCCCGGGATCGATAAGCTTTC 3'

L'oligonucléotide double brin ainsi obtenu a été ligaturé avec le plasmide pPB109, préalablement digéré par *Sal*I et traité avec la phosphatase alcaline, pour donner le plasmide pPB110 (4275 pb) (figure n° 3).

Ce plasmide contient les bras flanquants 5' (SacI-SalI 699 pb) et 3' (SalI-SmaI 668 pb) du site FHV-1 ORF5 ainsi qu'un site multiple de clonage permettant l'insertion d'une cassette d'expression.

### Exemple 6: Construction de la cassette d'expression pour le gène FIPV M (figure n ° 4)

Une réaction de RT-PCR a été réalisée avec l'ARN génomique de la souche FIPV 79-1146 et avec les oligonucléotides suivants:
JCA056 (40 mer) (SEQ ID N°18)
   5' TTTGAGCTCGCGGCCGCATGAAGTAATTTTGCTAATACTC 3'
JCA057 (27 mer) (SEQ ID N° 19)
   5' TTTGGTACCGTTTAGTTACACCATATG 3'
   en vue d'isoler précisément le gène codant pour la glycoprotéine de membrane (FIPV M) sous la forme d'une cassette SacI-KpnI. Après purification, le produit de RT-PCR de 823 pb a été digéré par *Kpn*I et *Sac*I pour isoler un fragment Kpnl-Sacl de 813 pb. Ce fragment a été ligaturé avec le vecteur pBS-SKII+, préalablement digéré avec *Kpn*I et *Sac*I, pour donner le vecteur pJCA080 (3668 pb). La séquence du gène M a été vérifiée par séquençage et a été trouvée identique à celle précédemment publiée (Vennema H. et al. Virology. 1991. 181. 327-335), séquence qui est incorporée par référence dans la présente demande.

Le plasmide pCMVβ (CLONTECH) a été digéré par *Eco*RI et *Not*I pour isoler le fragment EcoRI-NotI de 819 pb contenant la région promotrice du gène immediate-early du cytomégalovirus humain (fragment A). Le plasmide pJCA080 a été digéré par *Kpn*I et *Not*I pour isoler le fragment NotI-KpnI (gène FIPV M) de 804 pb (fragment B). Les fragments A et B ont alors été ligaturés avec le vecteur pGEM-7Zf + (Promega), préalablement digéré par *Eco*RI et *Kpn*I, pour donner le plasmide pPB104 (4614 pb).

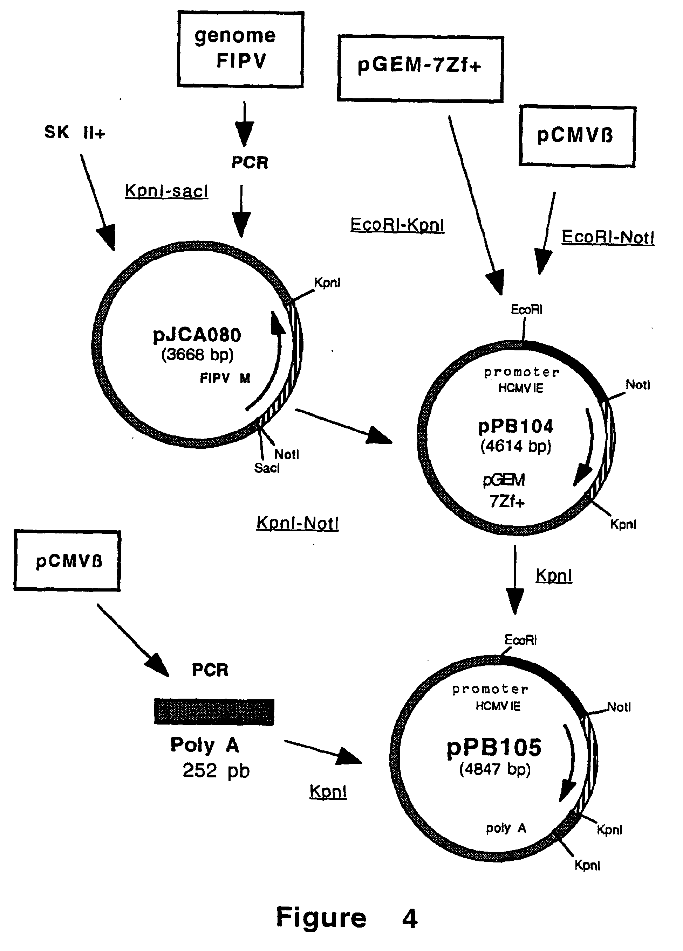

Une réaction PCR a été réalisée avec les oligonucléotides suivants:
PB088 (30 mer) (SEQ ID N° 20)
   5' TTGGGTACCGCCTCGACTCTAGGCGGCCGC 3'
PB089 (32 mer) (SEQ ID N° 21)
   5' TTGGGTACCGGATCCGAAAAAACCTCCCACAC 3'
et la matrice pCMVβ pour produire un fragment de 252 pb contenant le signal de polyadénylation du gène précoce du virus SV40. Ce fragment a été digéré par *Kpn*I pour isoler le fragment KpnI-KpnI de 233 pb (fragment B). Ce fragment a alors été ligaturé avec le plasmide pPB104, préalablement digéré par *Kpn*I et traité avec la phosphatase alcaline, pour donner le plasmide pPB105 (4847 pb) (figure n° 4). Ce plasmide contient une cassette d'expression promoteur HCMV-IE - gène FIPV M - polyA SV40 mobilisable par digestion *Apa*I*-Cla*I ou *Apa*I-*Hin*dIII*.*

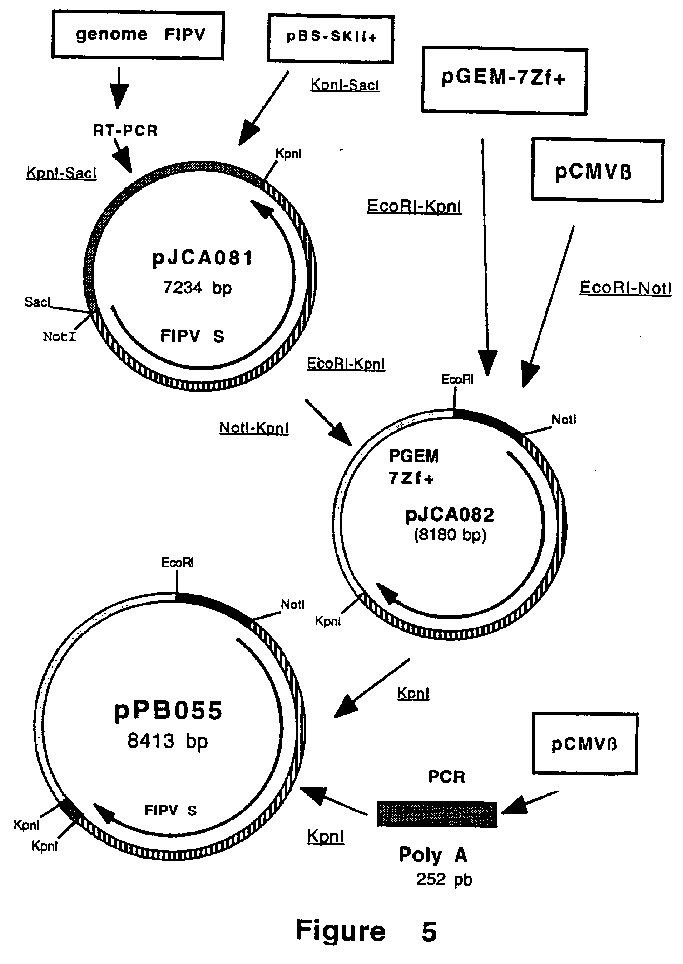

### Exemple 7: Construction de la cassette d'expression pour le gène FIPV S (figure n° 5)

Une réaction de RT-PCR a été réalisée avec l'ARN génomique de la souche FIPV 79-1146 et avec les oligonucléotides suivants:
JCA058 (39 mer) (SEQ ID N° 22)
   5'TTTGAGCTCGCGGCCGCATGATTGTGCTCGTAACTTGCC 3'
JCA059 (38 mer) (SEQ ID N° 23)
   5'TTTGGTACCGTTTAGTGGACATGCACTTTTTCAATTGG 3'
en vue d'isoler précisément le gène codant pour la glycoprotéine spicule (ou "spike" ou encore désignée ci-après "S") (FIPV S). Après purification, le produit de RT-PCR de 4387 pb a été digéré par *Kpn*I et *Sac*I pour isoler un fragment KpnI-SacI de 4375 pb. Ce fragment a été ligaturé avec le vecteur pBS-SKII+, préalablement digéré avec *Kpn*I et *Sac*I, pour donner le vecteur pJCA081 (7234 pb). La séquence du gène S a été vérifiée par séquençage et a été trouvée identique à celle précédemment publiée (de Groot R. et al. J. Gen. Virol. 1987. 68. 2639-2646), séquence qui est incorporée par référence dans la présente demande.

Le plasmide pCMVβ a été digéré par *Eco*RI et *Not*I pour isoler le fragment EcoRl-Notl de 819 pb contenant la région promotrice du gène immediate-early du cytomégalovirus humain (fragment A). Le plasmide pJCA081 a été digéré par *Kpn*I et *Not*I pour isoler le fragment Notl-Kpnl (gène FIPV S) de 4372 pb (fragment B). Les fragments A et B ont alors été ligaturés avec le vecteur pGEM-7Zf +, préalablement digéré par *Eco*RI et *Kpnl,* pour donner le plasmide pJCA082 (8180 pb).

Une réaction PCR a été réalisée avec les oligonucléotides suivants: PB088 (SEQ ID N° 20) et PB089 (SEQ ID N° 21) (voir exemple 6) et la matrice pCMVβ pour produire un fragment de 252 pb contenant le signal de polyadénylation du gène précoce du virus SV40. Ce fragment a été digéré par *Kpn*I pour isoler le fragment KpnI-KpnI de 233 pb (fragment B).

Ce fragment a été ligaturé avec le plasmide pJCA082, préalablement digéré par *Kpn*I et traité avec la phosphatase alcaline, pour donner le plasmide pPB055 (8413 pb)(figure n° 5). Ce plasmide contient une cassette d'expression promoteur HCMV-IE - gène FIPV S - polyA SV40 mobilisable par digestion Apal-*Cla*I.

### Exemple 8: Construction du gène spike modifié (FIPV S*)

La séquence du gène FIPV S a été mutagénisée de manière à modifier les régions responsables de l'induction d'anticorps facilitants, sans changer les fonctions de la glycoprotéine S. Cette modification a déjà été décrite dans la demande de brevet FR-94 10379 (publication N° 2 724 385), incorporée ici par référence, et a été réalisée de la manière suivante:

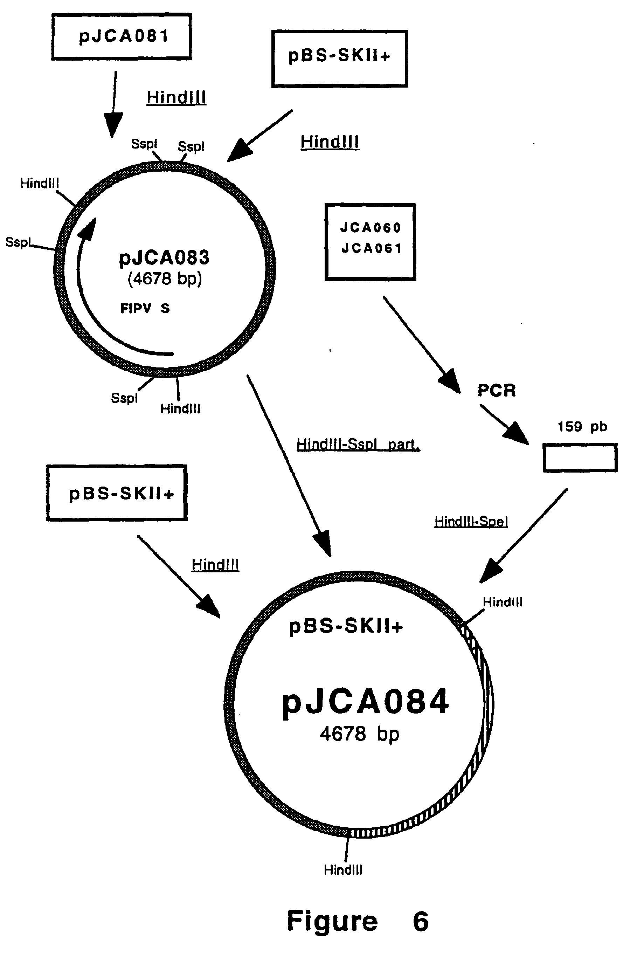

### 8.1. : Mutagénèse du site A1 (figure n° 6)

Le fragment central du gène FIPV S HindIII-HindIII de 1723 pb (nucléotides 1696 à 3418) a été cloné dans le vecteur pBS-SKII +, préalablement digéré par *Hin*dIII et traité avec la phophatase alcaline, pour donner le plasmide pJCA083 (4678 pb). Le site A1 est situé sur le sous-fragment HindIII-SspI (positions 1696 à 1845) de ce fragment.

Le site A1 a été mutagénisé par PCR en utilisant la stratégie suivante:
Les oligonucléotides suivants ont été synthétisés:
   JCA060 (95 mer) (SEQ ID N° 24)
      5'ATGAAGCTTAGTGGTTATGGTCAACCCATAGCCTCGACACTAAGTAACAT CACACTACCAATGCAGGATAACAATACTGTTGTGTACTGTATTCG 3'
   JCA061 (88 mer) (SEQ ID N° 25)
      5'AAAAATATTGTACCATAAAGAACTTTTGCAAGTGGAATGAACATAAACTG AGAATTGGTTAGAACGAATACAGTACACAACAGTATTG 3'
   JCA062 (20 mer) (SEQ ID N° 26)
      5' ATGAAGCTTAGTGGTTATGG 3'
   JCA063 (20 mer) (SEQ ID N° 27)
      5' AAAAATATTGTACCATAAAG 3'

Les oligonucléotides JCA060 et JCA061 ont été hybridés entre eux grâce à leur séquence complémentaire commune de 23 paires de bases. L'hybride ainsi obtenu a été utilisé, après élongation de ses extrémités 3', comme matrice pour une réaction PCR utilisant les oligonucléotides JCA062 et JCA063. Cette réaction d'amplification par PCR a permis d'obtenir un fragment de 159 pb. Ce fragment a été digéré par *Hin*dIII et *Ssp*I pour produire un fragment HindIII-SspI de 149 pb (fragment A). Ce fragment contient le site A1 modifié à deux positions (Val au lieu de Asp à la position 568 et Tyr au lieu de Asp à la position 591). Le plasmide pJCA083 a été digéré par *Hin*dIII et digéré partiellement par *Ssp*I pour isoler le fragment SspI-HindIII de 1569 pb (fragment B) par Geneclean (BIO101 Inc., La Jolla. Ca.).

Le vecteur pBS-SKII+ a été digéré par *Hin*dlll et traité avec la phosphatase alcaline pour produire le fragment C (2960 pb).

Les fragments A, B et C ont été ensuite ligaturés ensemble pour produire le plasmide pJCA084 (4678 pb) (figure n° 6). Ce plasmide contient le fragment HindIII-HindIII du gène FIPV S modifié pour deux acides aminés du site A1.

Le gène FIPV S peut être ensuite reconstitué en remplaçant le fragment Hindlll-Hindlll naturel (positions 1696 à 3418) par le fragment HindIII-HindIII contenu dans le plasmide pJCA084. Le gène complet FIPV S modifié au site A1 peut alors être utilisé pour des constructions de plasmides d'expression ou de virus recombinants.

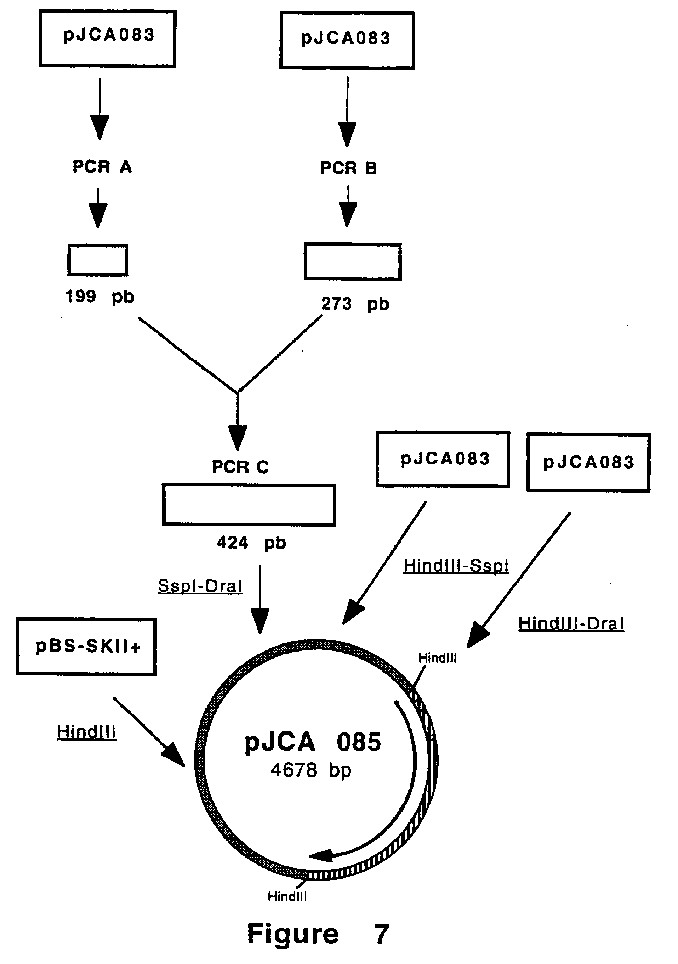

### 8.2. : Mutagénèse du site A2 (figure n° 7)

Les oligonucléotides suivants ont été synthétisés :
JCA064 (20 mer) (SEQ ID N° 28)
   5' GGACAATATTTTTAATCAAG 3'
JCA065 (36 mer) (SEQ ID N° 29)
   5' TTTAACAACCTGCTCATTGGTTCCTGTACGTGCAGC 3'
JCA066 (36 mer) (SEQ ID N° 30)
   5' AAGTTTTATGTTGCTGCACGTACAGGAACCAATGAG 3'
JCA067 (20 mer) (SEQ ID N° 31)
   5' ATCACTAACATTTTTAAAGC 3'

Une réaction PCR (PCR A) a été réalisée avec les oligonucléotides JCA064 et JCA065 et avec le plasmide pJCA083 commme matrice pour synthétiser un fragment PCR de 199 pb (fragment A).

Une réaction PCR (PCR B) a été réalisée avec les oligonucléotides JCA066 et JCA067 et avec le plasmide pJCA083 comme matrice pour donner un fragment PCR de 273 pb (fragment B).

Les fragments PCR A et B ont été hybridés entre eux grâce à leur région complémentaire de 46 pb et le produit de cette hybridation, après élongation des extrémités 3', a été amplifié par une réaction PCR (PCR C) avec les oligonucléotides JCA064 et JCA067 pour donner un fragment PCR de 424 pb. Ce fragment PCR a été digéré par *Ssp*I et *Dra*I pour donner le fragment de restriction SspI-DraI de 402 pb (fragment C).

Le plasmide pJCA083 a été digéré par *Hin*dIII et *Ssp*I pour isoler le fragment HindIII-SspI de 149 pb (fragment D).

Le plasmide pJCA083 a été digéré par *Hin*dIII et *Dra*I pour isoler le fragment de restriction Dral-Hindlll de 1170 pb (fragment E).

Le vecteur pBS-SKII + a été digéré par *Hin*dlll et traité avec la phosphatase alcaline pour donner le fragment F (2960 pb).

Les fragments C, D, E et F ont été ensuite ligaturés ensemble pour donner le plasmide pJCA085 (4678 pb) (figure n° 7). Le fragment central HindIII-HindIII 1723 pb du gène FIPV S contenu dans pJCA085 possède un site A2 modifié au niveau de 3 acides aminés (Tyr au lieu de Asp à la position 643, Gly au lieu de Arg à la position 649, et Lys au lieu de Arg à la position 656).

Le gène FIPV S peut être ensuite reconstitué en remplaçant le fragment Hindlll-Hindlll naturel (positions 1696 à 3418) par le fragment HindIII-HindIII contenu dans le plasmide pJCA085. Le gène complet FIPV S modifié au site A2 peut alors être utilisé pour des constructions de plasmides d'expression ou de virus recombinants.

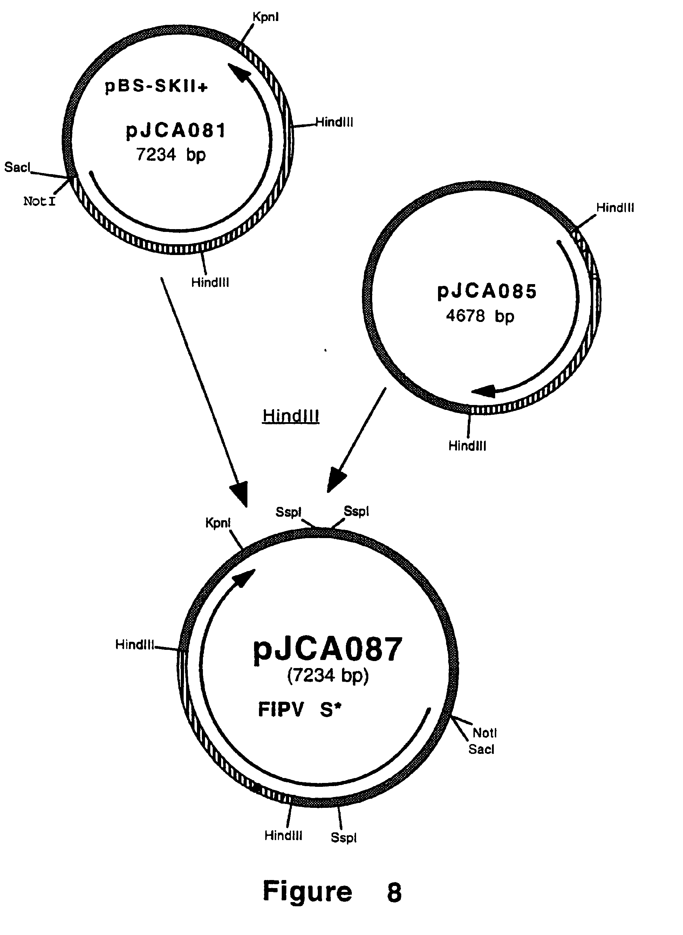

### 8.3. : Mutagénèse des sites A1 et A2 (figure n° 8)

Les fragments A (exemple 8.1), C et E (exemple 8.2) ont été ligaturés avec le vecteur pBS-SKII+, préalablement digéré par *Hind*III et traité avec la phosphatase alcaline, pour donner le plasmide pJCA085. Le fragment central HindIII-HindIII de 1723 pb du gène FIPV S contenu dans pJCA085 présente 2 changements d'acides aminés au niveau du site A1 (voir exemple 8.1) et 3 changements d'acides aminés au niveau du site A2 (voir exemple 8.2).

Le plasmide pJCA081 (exemple 7) a été digéré par *Hin*dIII pour isoler le fragment HindIII-HindIII de 5511 pb (fragment A). Le plasmide pJCA085 a été digéré par *Hind*III pour isoler le fragment HindIII-HindIII de 1723 pb (présentant 5 changements d'acides aminés par rapport à la séquence de la souche FIPV 79-1146) (fragment B). Les fragments A et B ont été ligaturés ensemble pour donner le plasmide pJCA087 (7234 pb) (figure n° 8). Ce plasmide contient le gène FIPV S modifié au niveau des sites A1 et A2 (= gène FIPV S*).

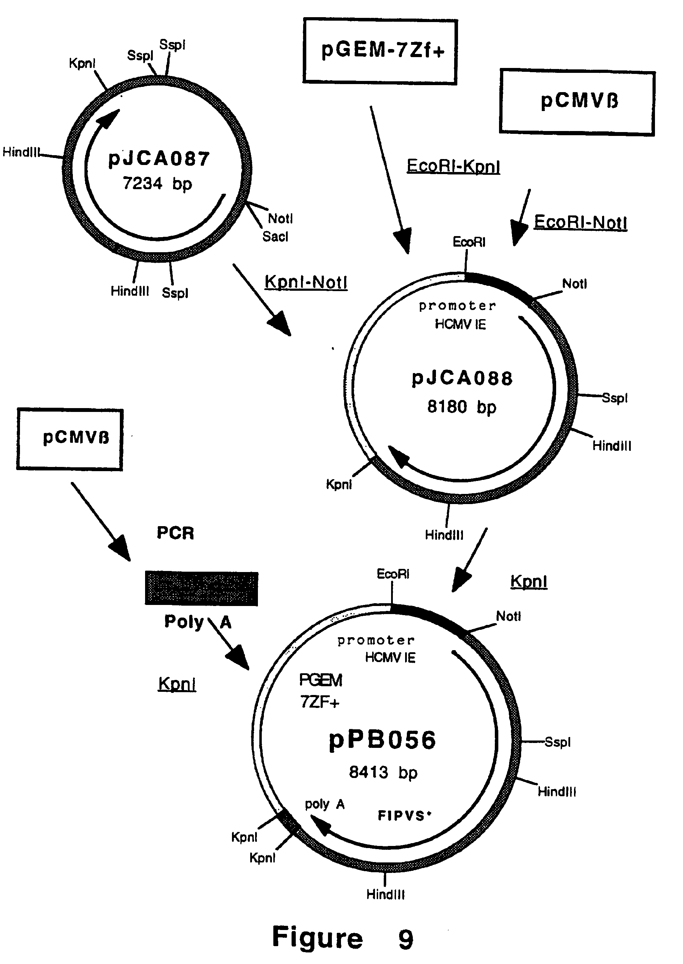

### 8.4. Construction de la cassette d'expression pour le gène FIPV S* (figure n°9)

Le plasmide pCMVβ a été digéré par *Eco*RI et *Not*I pour isoler le fragment EcoRl-Notl de 819 pb contenant la région promotrice du gène immediate-early du cytomégalovirus humain (fragment A). Le plasmide pJCA087 (exemple 8.3) a été digéré par *Kpn*I et *Not*I pour isoler le fragment Notl-Kpnl (gène FIPV S) de 4372 pb (fragment B). Les fragments A et B ont alors été ligaturés avec le vecteur pGEM-7Zf+, préalablement digéré par *Eco*RI et *Kpn*I*,* pour donner le plasmide pJCA088 (8180 pb).

Une réaction PCR a été réalisée avec les oligonucléotides suivants:
PB088 (SEQ ID N° 20) et PB089 (SEQ ID N° 21) (voir exemple 6) et la matrice pCMVβ pour produire un fragment de 252 pb contenant le signal de polyadénylation du gène précoce du virus SV40. Ce fragment a été digéré par *Kpn*I pour isoler le fragment Kpnl-Kpnl de 233 pb (fragment B).

Ce fragment a été ligaturé avec le plasmide pJCA088, préalablement digéré par *Kpn*I et traité avec la phosphatase alcaline, pour donner le plasmide pPB056 (8413 pb) (figure n° 9). Ce plasmide contient une cassette d'expression promoteur HCMV-IE - gène FIPV S* - polyA SV40 mobilisable par digestion *Apa*I-*Cla*I.

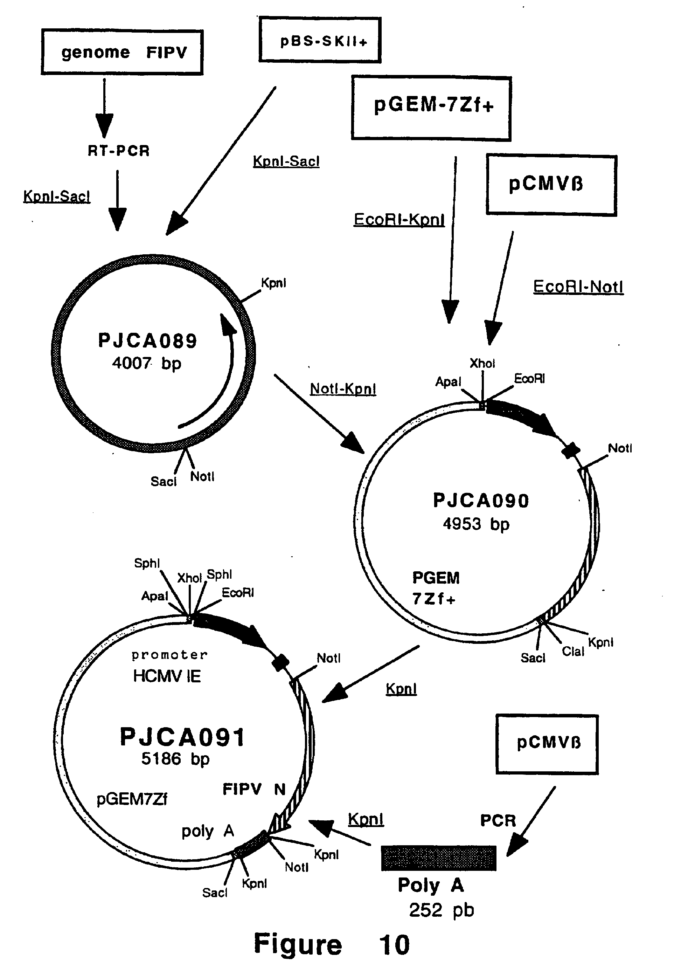

### Exemple 9: Construction de la cassette d'expression pour le gène FIPV N (figure n° 10)

Une réaction de RT-PCR a été réalisée avec l'ARN génomique de la souche FIPV 79-1146 et avec les oligonucléotides suivants:
JCA068 (37 mer) (SEQ ID N° 32)
   5' TTTGAGCTCGCGGCCGCATGGCCACACAGGGACAACG 3'
JCA069 (33 mer) (SEQ ID N° 33)
   5' TTTGGTACCGTTTAGTTCGTAACCTCATCAATC 3'
pour isoler précisément le gène codant pour la protéine de nucléocapside "N" (FIPV N). Après purification, le produit de RT-PCR de 1161 pb a été digéré par *Kpn*I et *Sac*I pour isoler un fragment Sacl-Kpnl de 1148 pb. Ce fragment a été ligaturé avec le vecteur pBS-SKII +, préalablement digéré avec *Kpn*I et *Sac*I, pour donner le vecteur pJCA089 (4007 pb). La séquence du gène N a été vérifiée par séquençage et a été trouvée identique à celle précédemment publiée (Vennema H. et al. Virology. 1991. 181. 327-335), séquence qui est incorporée par référence dans la présente demande.

Le plasmide pCMVβ (CLONTECH) a été digéré par *Eco*RI et *Not*I pour isoler le fragment EcoRI-NotI de 819 pb contenant la région promotrice du gène immediate-early du cytomégalovirus humain (fragment A). Le plasmide pJCA089 a été digéré par *Kpn*I et *Not*I pour isoler le fragment Notl-Kpnl (gène FIPV N) de 1137 pb (fragment B). Les fragments A et B ont été ligaturés avec le vecteur pGEM-7Zf+, préalablement digéré par *Eco*RI et *Kpn*I*,* pour donner le plasmide pJCA090 (4953 pb).

Une réaction PCR a été réalisée avec les oligonucléotides suivants:
PB088 (SEQ ID N° 20) et PB089 (SEQ ID N° 21) (exemple 6) et la matrice pCMVβ pour produire un fragment de 252 pb contenant le signal de polyadénylation du gène précoce du virus SV40. Ce fragment a été digéré par *Kpn*I pour isoler le fragment Kpnl-Kpnl de 233 pb (fragment B). Ce fragment a alors été ligaturé avec le plasmide pJCA090, préalablement digéré par *Kpn*I et traité avec la phosphatase alcaline, pour donner le plasmide pJCA091 (5186 pb) (figure n° 10). Ce plasmide contient une cassette d'expression promoteur HCMV-IE - gène FIPV N - polyA SV40 mobilisable par digestion *Apa*I-*Cla*I ou *Apa*I-*Hin*dIII.

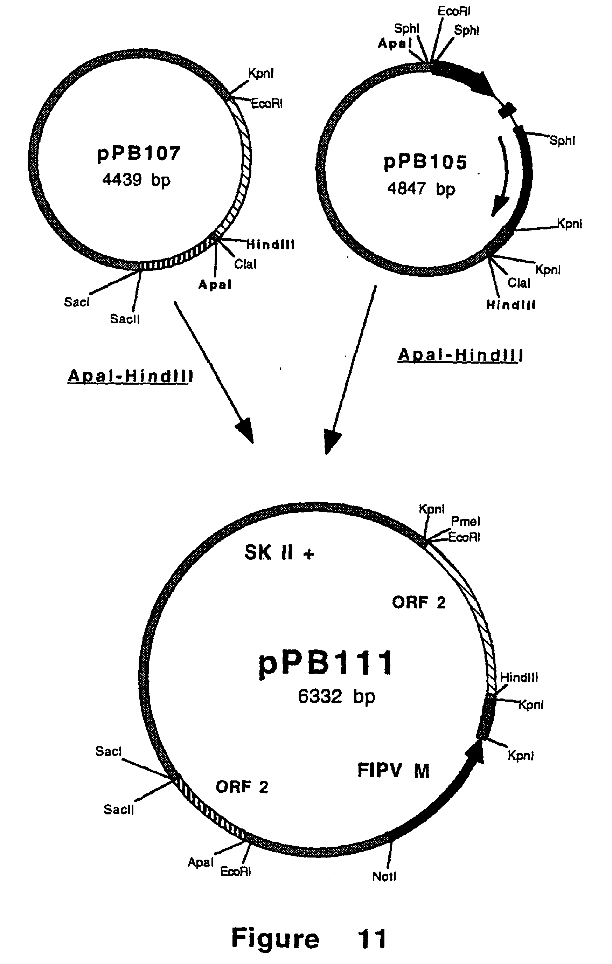

### Exemple 10: Construction du plasmide donneur pPB111 et isolement de vFHV01 (figure n°11)

Le plasmide pPB105 (exemple 6, figure n°4) a été digéré par *Apa*I et *Hin*dIII pour isoler le fragment ApaI-HindIII de 1925 pb (fragment A). Le plasmide pPB107 (exemple 4, figure n° 2) a été digéré par *Apal* et *Hin*dIII pour isoler le fragment ApaI-HindIII de 4419 pb (fragment B). Les fragments A et B ont été ligaturés ensemble pour donner le plasmide pPB111 (6332 pb) (figure n° 11).

Ce plasmide contient la cassette d'expression HCMV-IE/gène FIPV M/polyA SV40 dans le site FHV ORF2.

Le plasmide pPB111 a été linéarisé par digestion avec *Pme*I, extrait avec un mélange phénol-chloroforme, précipité avec de l'éthanol absolu, puis repris dans de l'eau stérile.

Des cellules CRFK, formant un tapis cellulaire bien établi en boîte de Petri (Corning 4,5 cm de diamètre) ont ensuite été transfectées avec le mélange suivant:

1 µg de plasmide pPB111 linéarisé + 5 µg d'ADN viral de FHV-1 dans 300 µl de milieu MEM et 100 µg de LipofectAMINE (Gibco-BRL Cat# 18324-012) dilués dans 300 µl de milieu (volume final du mélange = 600 µl). Ces 600 µl ont été ensuite dilués dans 3 ml (volume final) de milieu MEM et étalés sur 3.10⁶ cellules CRFK. Le mélange a été laissé en contact avec les cellules pendant 5 heures, puis éliminé et remplacé par 5 ml de milieu de culture. Les cellules ont alors été laissées en culture pendant 24 heures à + 37°C. Après 24 heures à 48 heures de culture, 1 ml de surnageant de culture a été récolté et plusieurs dilutions de ce surnageant ont été utilisées pour infecter de nouvelles cellules CRFK (cultivées en boîte de Petri (Corning 4,5 cm de diamètre) de manière à obtenir des plages isolées, chaque boîte étant infectée avec 1 ml d'une dilution du surnageant initial. Après un contact d' 1 heure à 37°C, le milieu d'infection a été éliminé et remplacé par 5 ml de milieu MEM à 1 % d'agarose, maintenu en surfusion à 42°C. Lorsque l'agarose a été solidifiée, les boîtes ont été incubées 48 heures à 37°C en étuve CO₂ jusqu'à apparition de plages. La couche d'agarose a alors été éliminée et un transfert des plages virales a été réalisé sur une membrane stérile de nitrocellulose de même diamètre que la boîte de Petri ayant servi à la culture. Cette membrane a été elle-même transférée sur une autre membrane de nitrocellulose de manière à obtenir une "copie" inversée du premier transfert. Les plages transférées sur cette dernière copie ont été alors hybridées, selon les techniques usuelles connues de l'homme de l'art, avec un fragment du gène FIPV M marqué à la digoxigénine (DNA Labelling Kit, Boehringer Mannheim, CAT # 1175033). Après hybridation, lavages et mise en contact avec le substrat de révélation, la membrane de nitrocellulose a été mise en contact avec un film autoradiographique. Les images d'hybridation positive sur cette membrane ont indiqué quelles étaient les plages qui contenaient des virus FHV recombinants ayant inséré la cassette FIPV M. Les plages correspondant à ces plages positives ont été découpées stérilement sur la première membrane de nitrocellulose, placées dans un tube Eppendorf contenant 0,5 ml de milieu MEM et soniquées pour libérer les virions de la membrane. Le milieu contenu dans le tube Eppendorf a été ensuite dilué en milieu MEM et les dilutions ainsi obtenues ont servi à infecter de nouvelles cultures de cellules CRFK. Un virus recombinant, contenant la cassette HCMV-IE/FIPV M/polyA insérée dans le site ORF2, pur à 100 % a été ainsi isolé après 3 cycles de purification et a été appelé vFHV01. L'homologie de la recombinaison a été vérifiée par PCR en utilisant des oligonucléotides situés de part et d'autre du site d'insertion. L'absence de remaniement sur le génome du virus recombinant vFHV01, ailleurs que dans la région de recombinaison, a été vérifiée par la technique du Southern blot.

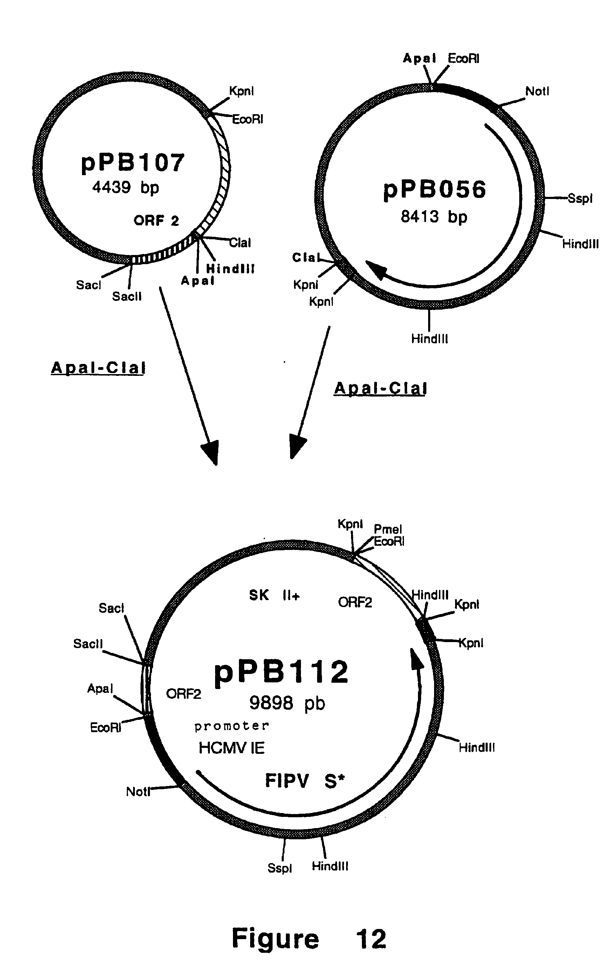

### Exemple 11: Construction du plasmide donneur pPB112 et isolement de vFHV02 (figure n° 12)

Le plasmide pPB056 (exemple 8.4, figure n° 9) a été digéré par *Apa*I et *Cla*I pour isoler le fragment ApaI-ClaI de 5466 pb (fragment A). Le plasmide pPB107 (exemple 4, figure n° 2) a été digéré par *Apal* et *Cla*I pour isoler le fragment Apal-Clal de 4426 pb (fragment B). Les fragments A et B ont été ligaturés ensemble pour donner le plasmide pPB112 (9898 pb) (figure n° 12). Ce plasmide contient la cassette d'expression (HCMV-IE/gène FIPV S*/polyA SV40) dans le site FHV-1 ORF2.

Des cellules CRFK ont été transfectées avec un mélange de plasmide pPB112 (linéarisé par *Pme*I) et d'ADN viral de FHV-1 comme décrit dans l'exemple 10. Une plage virale positive pour la cassette HCMV-IE/gène FIPV S* a été purifiée comme décrit dans l'exemple 10, mais en utilisant une sonde homologue FIPV S*, et amplifiée pour donner le virus recombinant vFHV02.

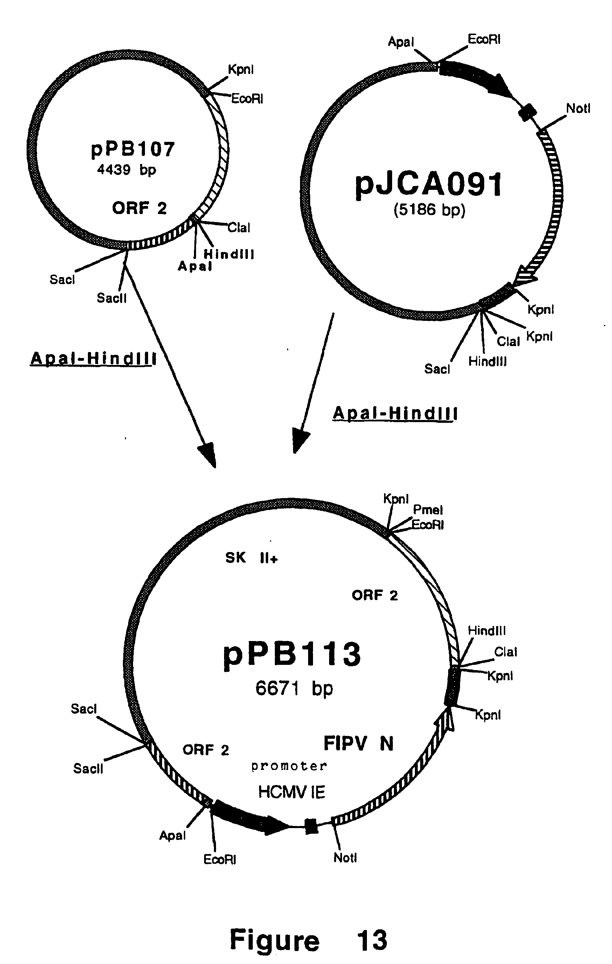

### Exemple 12: Construction du plasmide donneur pPB113 et isolement de vFHV03 (figure n° 13)

Le plasmide pJCA091 (exemple 9, figure n° 10) a été digéré par *Apal* et *Hin*dIII pour isoler le fragment ApaI-HindIII de 2244 pb (fragment A). Le plasmide pPB107 (exemple 4, figure n° 2) a été digéré par *Apa*I et *Hind*III pour isoler le fragment ApaI-HindIII de 4419 pb (fragment B). Les fragments A et B ont été ligaturés ensemble pour donner le plasmide pPB113 (6671 pb) (figure n°13).

Ce plasmide contient la cassette d'expression (HCMV-IE/gène FIPV N/polyA SV40) dans le site FHV-1 ORF2.

Des cellules CRFK ont été transfectées avec un mélange de plasmide pPB113 (linéarisé par *Pme*I) et d'ADN viral de FHV-1 comme décrit dans l'exemple 10. Une plage virale positive pour la cassette HCMV-IE/gène FIPV N a été purifiée comme décrit dans l'exemple 10, mais en utilisant une sonde homologue FIPV N, et amplifiée pour donner le virus recombinant vFHV03.

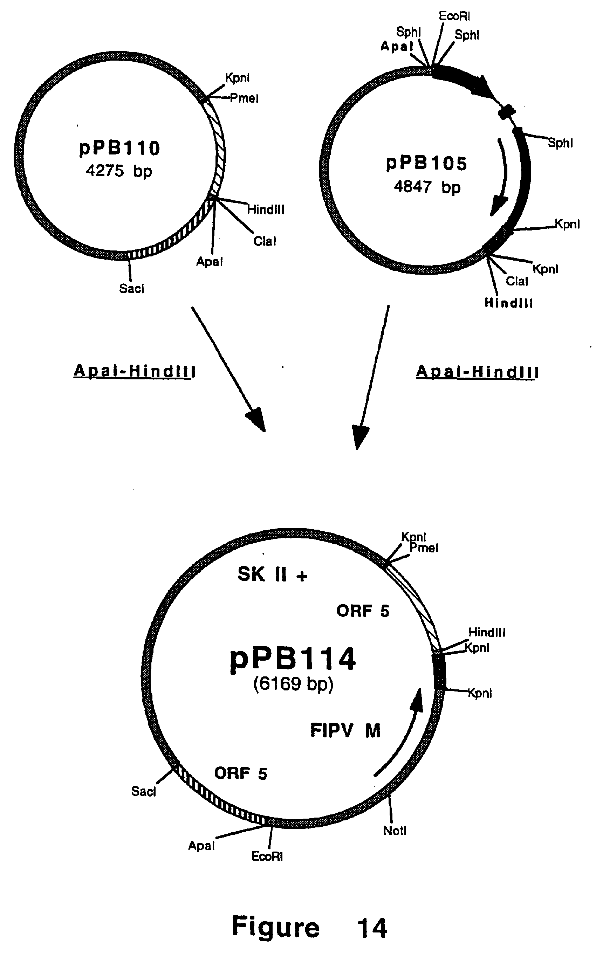

### Exemple 13: Construction du plasmide donneur pPB114 et isolement de vFHV04 (figure n° 14)

Le plasmide pPB105 (exemple 6, figure n° 4) a été digéré par *Apa*I et *Hin*dIII pour isoler le fragment ApaI-HindIII de 1925 pb (fragment A). Le plasmide pPB110 (exemple 5, figure n° 3) a été digéré par *Apa*I et *Hin*dIII pour isoler le fragment ApaI-HindIII de 4256 pb (fragment B). Les fragments A et B ont été ligaturés ensemble pour donner le plasmide pPB114 (6169 pb) (figure n° 14). Ce plasmide contient la cassette d'expression (HCMV-IE/gène FIPV M/polyA SV40) dans le site FHV-1 ORF5.

Des cellules CRFK ont été transfectées avec un mélange de plasmide pPB114 (linéarisé par *Pme*I) et d'ADN viral de FHV-1 comme décrit dans l'exemple 10. Une plage virale positive pour la cassette HCMV-IE/gène FIPV M a été purifiée comme décrit dans l'exemple 10 et amplifiée pour donner le virus recombinant vFHV04.

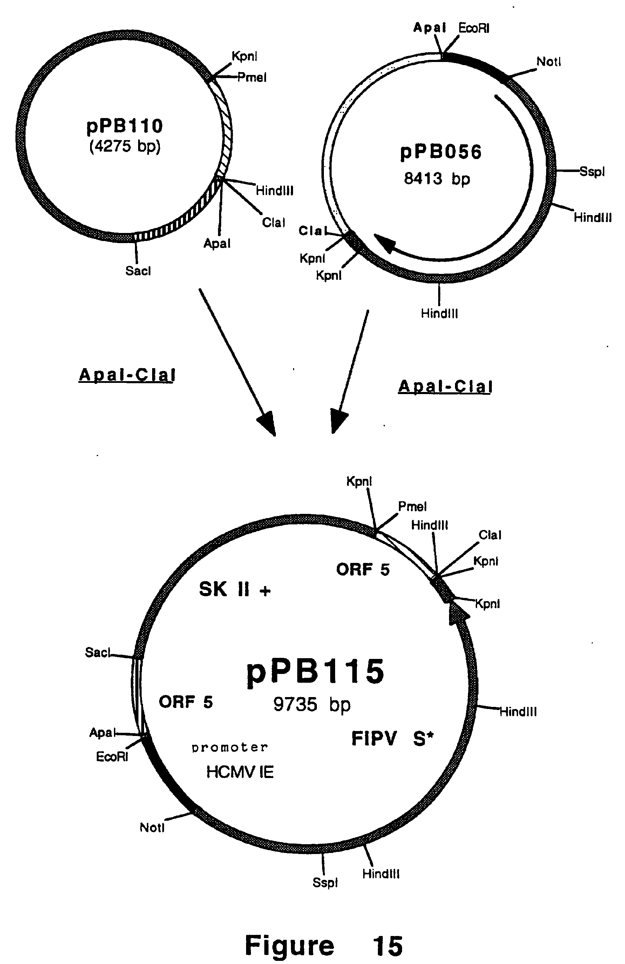

### Exemple 14: Construction du plasmide donneur pPB115 et isolement de vFHV05 (figure n° 15)

Le plasmide pPB056 (exemple 8.4, figure n° 9) a été digéré par *Apa*I et *Cla*I pour isoler le fragment ApaI-ClaI de 5466 pb (fragment A). Le plasmide pPB110 (exemple 5, figure n° 3) a été digéré par *Apa*I et *Cla*I pour isoler le fragment ApaI-ClaI de 4263 pb (fragment B). Les fragments A et B ont été ligaturés ensemble pour donner le plasmide pPB115 (9735 pb) (figure n° 5). Ce plasmide contient la cassette d'expression (HCMV-IE/gène FIPV S*/polyA SV40) dans le site FHV-1 ORF5.

Des cellules CRFK ont été transfectées avec un mélange de plasmide pPB115 (linéarisé par *Pme*I) et d'ADN viral de FHV-1 comme décrit dans l'exemple 10. Une plage virale positive pour la cassette HCMV-IE/gène FIPV S* a été purifiée comme décrit dans l'exemple 10, mais en utilisant une sonde homologue FIPV S*, et amplifiée pour donner le virus recombinant vFHV05.

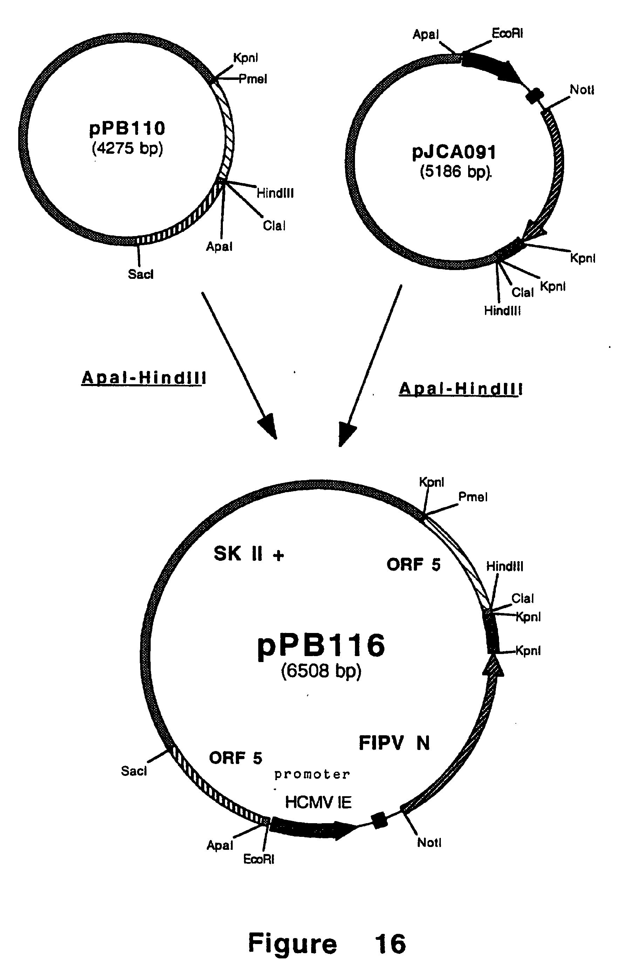

### Exemple 15: Construction du plasmide donneur pPB116 et isolement de vFHV06 (figure n° 16)

Le plasmide pJCA091 (exemple 9, figure n° 10) a été digéré par *Apal* et *Hin*dIII pour isoler le fragment ApaI-HindIII de 2244 pb (fragment A). Le plasmide pPB110 (exemple 5, figure n° 3) a été digéré par *Apa*I et *Hin*dIII pour isoler le fragment ApaI-HindIII de 4256 pb (fragment B). Les fragments A et B ont été ligaturés ensemble pour donner le plasmide pPB116 (6508 pb). Ce plasmide contient la cassette d'expression (HCMV-IE/gène FIPV N/polyA SV40) dans le site FHV-1 ORF5.

Des cellules CRFK ont été transfectées avec un mélange de plasmide pPB116 (linéarisé par *Pme*I) et d'ADN viral de FHV-1 comme décrit dans l'exemple 10. Une plage virale positive pour la cassette HCMV-IE/gène FIPV N a été purifiée comme décrit dans l'exemple 10, mais en utilisant une sonde homologue FIPV N, et amplifiée pour donner le virus recombinant vFHV06.

### Exemple 16: Construction de plasmides donneurs pour l'insertion de cassettes d'expression dans le site ORF2 de FHV-1

Selon la même stratégie que celle décrite plus haut pour l'insertion de cassettes d'expression (gènes placés sous le contrôle des promoteurs HCMV-IE ou MCMV-IE ou double promoteur MCMV-IE/RNA 1.8 kpb) dans le site ORF2, il est possible de construire des herpèsvirus félins recombinants exprimant à un niveau élevé des immunogènes du calicivirus félin (FCV), du virus de la panleucopénie féline (FPV), du virus de la leucémie féline (FeLV), du virus de l'immunodéficience féline (FIV), ou d'autres pathogènes félins, ou encore des cytokines félines.

### Exemple 17: Construction de plasmides donneurs pour l'insertion de cassettes d'expression dans le site ORF5 de FHV-1

Selon la même stratégie que celle décrite plus haut pour l'insertion de cassettes d'expression (gènes placés sous le contrôle des promoteurs HCMV-IE ou MCMV-IE ou double promoteur MCMV-IE/RNA 1.8 kpb) dans le site ORF5, il est possible de construire des herpèsvirus félins recombinants exprimant à un niveau élevé des immunogènes des virus FCV, FPV, FeLV, FIV, ou d'autres pathogènes félins, ou encore des cytokines félines.

### Exemple 18: Production de vaccins

Pour produire un vaccin, les virus recombinants obtenus selon l'invention sont cultivés sur cellules CRFK. La récolte du virus recombinant se fait lorsque l'effet cytopathique est complet. Les cellules lysées et le surnageant de culture sont récoltés. Après clarification du lysat cellulaire pour éliminer les débris cellulaires, la solution virale est titrée. La solution virale est ensuite diluée dans une solution stabilisatrice pour la lyophilisation, répartie à raison d'une dose vaccinale (10² DICC50 à 10⁷ DICC50) par flacon, et enfin lyophilisée.

## Revendications

1. Vaccin vivant recombinant comprenant, comme vecteur, un herpèsvirus félin comprenant et exprimant au moins une séquence nucléotidique codant pour un polypeptide, cette séquence étant insérée dans les sites ORF5 et/ou ORF2 de FHV, sites qui, dans la site FHV-1 CO, présentent les séquences nucléotiques de positions respectives 5869-7113 et 1655-2596 sur la SEQ ID NO: 1.

2. Vaccin vivant recombinant selon la revendication 1 , **caractérisé en ce que** la ou les séquences nucléotidiques sont insérées dans les sites ORF5 et/ou ORF2 par insertion simple, ou après délétion totale ou partielle de ces sites.

3. Vaccin vivant recombinant selon la revendication 1, **caractérisé en ce que** la ou les séquences nucléotidiques sont insérées dans l'un des sites ORF5 ou ORF2, et qu'une délétion est réalisée dans l'autre de ces sites.

4. Vaccin vivant recombinant selon l'une quelconque des revendications 1 à 3, **caractérisé en ce que**, pour exprimer la séquence insérée, le vecteur comprend un promoteur eucaryote fort.

5. Vaccin vivant recombinant selon la revendication 4, **caractérisé en ce que** le promoteur fort est un promoteur CMV immediate-early, de préférence le promoteur CMV immediate-early murin ou humain.

6. Vaccin vivant recombinant selon l'une quelconque des revendications 1 à 5, **caractérisé en ce qu'**il comprend au moins deux séquences nucléotidiques insérées dans le site ORF5 ou ORF2 sous le contrôle de promoteurs eucaryotes différents.

7. Vaccin vivant recombinant selon la revendication 6, **caractérisé en ce que** les promoteurs eucaryotes sont des promoteurs CMV immediate-early d'origines animales différentes.

8. Vaccin vivant recombinant selon la revendication 6, **caractérisé en ce qu'**il comprend une première séquence nucléotidique associée au promoteur CMV immediate early et une autre séquence nucléotidique associée à un autre promoteur, les deux promoteurs ayant leurs extrémités 5' adjacentes.

9. Vaccin vivant recombinant selon l'une quelconque des revendications 1 à 8, **caractérisé en ce qu'**il comprend une séquence nucléotidique codant pour un polypeptide antigénique, cette séquence étant insérée dans les sites ORF5 et/ou ORF2.

10. Vaccin vivant recombinant selon la revendication 9, **caractérisé en ce qu'**il comprend une séquence nucléotidique codant pour un polypeptide antigénique d'un agent pathogène félin, cette séquence étant insérée dans les sites ORF5 et/ou ORF2.

11. Vaccin vivant recombinant selon la revendication 10, **caractérisé en ce qu'**il comprend une séquence codant pour un antigène choisi parmi le groupe des antigènes du virus de la péritonite infectieuse féline, du virus de la leucémie féline, du virus de l'immunodéficience féline, du virus de la panleucopénie infectieuse féline et du calicivirus félin, cette séquence étant insérée dans les sites ORF5 et/ou ORF2.

12. Vaccin vivant recombinant selon la revendication 10, **caractérisé en ce qu'**il comprend une séquence nucléotidique, choisie parmi les séquences nucléotidiques codant pour les polypeptides M, S modifié ou N de FIPV, cette séquence étant insérée dans les sites ORF5 et/ou ORF2.

13. Vaccin vivant recombinant selon la revendication 10, **caractérisé en ce qu'**il comprend au moins une séquence nucléotidique choisie parmi le groupe des séquences correspondant aux antigènes env, gag, pol du virus de la leucémie féline, aux antigènes env, gag, pol du virus de l'immunodéficience féline, à l'antigène de capside VP2 du virus de la panleucopénie infectieuse féline, à l'antigène de capside du calicivirus félin, aux antigènes M, S modifié, N, 7b, polymérase du virus de la péritonite infectieuse féline.

14. Vaccin vivant recombinant selon l'ensemble des revendications 8 et 12, **caractérisé en ce que** la séquence nucléotidique insérée sous le contrôle du promoteur CMV immediate early est une séquence nucléotidique codant pour le polypeptide M, N ou S modifié du virus FIPV et **en ce que** la séquence nucléotidique insérée sous le contrôle du promoteur associé est une séquence nucléotidique codant pour un antigène d'une autre maladie féline.

15. Vaccin vivant recombinant selon l'une quelconque des revendications 1 à 14, **caractérisé en ce qu'**il comprend une séquence nucléotidique codant pour un polypeptide immunomodulateur, cette séquence étant insérée dans les sites ORF5 et/ou ORF2.

16. Vaccin vivant recombinant selon la revendication 15, **caractérisé en ce que** cette séquence nucléotidique est choisie parmi le groupe des séquences codant pour des cytokines.

17. Formule de vaccin multivalent comprenant, en mélange ou à mélanger, au moins deux vaccins vivants recombinants tels que définis dans l'une quelconque des revendications 1 à 16, ces vaccins comprenant des séquences insérées différentes.

18. Fragment d'ADN comprenant au moins une séquence choisie dans le groupe constitué par :
- la séquence définie par les positions 1 à 8193 sur la SEQ ID NO:1 ;
- la séquence du site ORF2 définie par les positions 1655 à 2596 sur la SEQ ID NO:1 ;
- la séquence du site ORF5 définie par les positions 5869 à 7113 sur la SEQ ID NO:1 ;
- les séquences flanquantes de 100 à 800 bp situées en amont et en aval du site ORF2 sur la SEQ ID NO:1 ;
- les séquences flanquantes de 100 à 800 bp situées en amont et en aval du site ORF5 sur la SEQ ID NO:1

19. Utilisation d'un vaccin selon l'une quelconque des revendications 1-16 ou d'une formule de vaccin selon la revendication 17 pour la préparation d'un vaccin pour chats destiné à une administration par voie parentérale ou locale.

20. Utilisation selon la revendication 19, dans laquelle ledit vaccin pour chats est destiné à une administration par voie oronasale.

21. Utilisation selon la revendication 19 ou 20, dans laquelle la dose vaccinale est 10² DICC50 à 10⁷ DICC50.

22. Utilisation selon l'une quelconque des revendications 19-21, dans laquelle ledit vaccin pour chats est destiné à une administration en une seule fois.

## Claims

1. A live recombinant vaccine comprising as a vector a feline herpesvirus (FHV) comprising and expressing at least one nucleotide sequence coding for a polypeptide, said sequence being inserted into the ORF5 and/or ORF2 sites of FHV, which sites in the FHV-1 CO site, have the nucleotic sequences in respective positions 5869-7113 and 1665-2596 on the SEQ ID NO:1.

2. A live recombinant vaccine according to claim 1 **characterised in that** the nucleotide sequence or sequences are inserted into the ORF5 and/or ORF2 sites by simple insertion or after total or partial deletion of said sites.

3. A live recombinant vaccine according to claim 1 **characterised in that** the nucleotide sequence or sequences are inserted into one of the ORF5 or ORF2 sites and that a deletion is effected in the other of said sites.

4. A live recombinant vaccine according to any one of claims 1 to 3 **characterised in that** to express the inserted sequence the vector comprises a strong eukaryotic promoter.

5. A live recombinant vaccine according to claim 4 **characterised in that** the strong promoter is a CMV intermediate-early promoter, preferably the murine or human CMV intermediate-early promoter.

6. A live recombinant vaccine according to any one of claims 1 to 5 **characterised in that** it comprises at least two nucleotide sequences inserted into the ORF5 or ORF2 site under the control of different eukaryotic promoters.

7. A live recombinant vaccine according to claim 6 **characterised in that** the eukaryotic promoters are CMV intermediate-early promoters of different animal origins.

8. A live recombinant vaccine according to claim 8 **characterised in that** it comprises a first nucleotide sequence associated with the CMV intermediate-early promoter and another nucleotide sequence associated with another promoter, the two promoters having their 5' ends adjacent.

9. A live recombinant vaccine according to any one of claims 1 to 8 **characterised in that** it comprises a nucleotide sequence coding for an antigenic polypeptide, said sequence being inserted into the ORF5 and/or ORF2 sites.

10. A live recombinant vaccine according to claim 9 **characterised in that** it comprises a nucleotide sequence coding for an antigenic polypeptide of a feline pathogenic agent, said sequence being inserted into the ORF5 and/or ORF2 sites.

11. A live recombinant vaccine according to claim 10 **characterised in that** it comprises a sequence coding for an antigen selected from the group of the antigens of the feline infectious peritonitis virus, the feline leukaemia virus, the feline immunodeficiency virus, the feline infectious panleukopenia virus and the feline calicivirus, said sequence being inserted into the ORF5 and/or ORF2 sites.

12. A live recombinant vaccine according to claim 10 **characterised in that** it comprises a nucleotide sequence selected from the nucleotide sequences coding for the M, modified S or N polypeptides of FIPV, said sequence being inserted into the ORF5 and/or ORF2 sites.

13. A live recombinant vaccine according to claim 10 **characterised in that** it comprises at least one nucleotide sequence selected from the group of sequences corresponding to the env, gag, pol antigens of the feline leukaemia virus, the env, gag, pol antigens of the feline immunodeficiency virus, the VP2 capsid antigen of the feline infectious panleukopenia virus, the capsid antigen of feline calicivirus, the M, modified S, N, 7b and polymerase antigens of the feline infectious peritonitis virus.

14. A live recombinant vaccine according to claims 8 and 12 in combination **characterised in that** the nucleotide sequence inserted under the control of the CMV intermediate-early promoter is a nucleotide sequence coding for the M, N or modified S polypeptide of the FIPV virus and that the nucleotide sequence inserted under the control of the associated promoter is a nucleotide sequence coding for an antigen of another feline disease.

15. A live recombinant vaccine according to any one of claims 1 to 14 **characterised in that** it comprises a nucleotide sequence coding for an immunomodulator polypeptide, said sequence being inserted in the ORF5 and/or ORF2 sites.

16. A live recombinant vaccine according to claim 15 **characterised in that** said nucleotide sequence is selected from the group of sequences coding for cytokines.

17. A multivalent vaccine formula comprising as a mixture or to be mixed at least two live recombinant vaccines as defined in any ane of claims 1 to 16, said vaccines comprising different inserted sequences.

18. A DNA fragment comprising at least one sequence selected from the group formed by
- the sequence defined by positions 1 to 8193 on the SEQ ID NC:1;
- the sequence of the ORF2 site defined by the positions 1655 to 2596 on the SEQ ID NO:1;
- the sequence of the ORF5 site defined by the positions 5869 to 7113 on the SEQ ID NO:1;
- the flanking sequences of 100 to 800 bp disposed upstream and downstream of the ORF2 site on the SEQ ID NO:1;
- the flanking sequences of 100 to 800 bp disposed upstream and downstream of the ORF5 site on the SEQ ID NO:1;
- the variants of said sequences corresponding to the equivalent sequences of the other strains of FHV.

19. Use of a vaccine according to any one of claims 1 to 16 or a vaccine formula according to claim 17 for the preparation of a vaccine for cats intended for parenteral or local administration.

20. Use according to claim 19 wherein said vaccine for cats is intended for oronasal administration.

21. Use according to claim 19 or claim 20 wherein the vaccine dose is 10² DICC50 to 10⁷ DICC50.

22. Use according to any one of claims 19 to 21 wherein said vaccine for cats is intended for a one-time administration.

## Patentansprüche

1. Rekombinanter Lebendimpfstoff, der als Vektor einen felinen Herpesvirus umfasst, der mindestens eine ein Polypeptid codierende Nucleotidsequenz umfasst und exprimiert, wobei die Sequenz in die ORF5- und/oder ORF2-Region von FHV insertiert ist, Regionen, die in der Region FHV-1 CO die Nucleotidsequenzen der Positionen 5869-7113 bzw. 1655-2596 der SEQ ID NO:1 aufweisen.

2. Rekombinanter Lebendimpfstoff nach Anspruch 1, **dadurch gekennzeichnet, dass** die Nucleotidsequenz oder die Nucleotidsequenzen durch einfache Insertion in die ORF5- und/oder ORF2-Region oder nach vollständiger oder teilweiser Deletion dieser Regionen insertiert werden.

3. Rekombinanter Lebendimpfstoff nach Anspruch 1, **dadurch gekennzeichnet, dass** die Nucleotidsequenz oder die Nucleotidsequenzen in einer der Regionen ORF5 oder ORF2 insertiert werden und dass in der anderen Region eine Deletion durchgeführt wird.

4. Rekombinanter Lebendimpfstoff nach einem der Ansprüche 1 bis 3, **dadurch gekennzeichnet, dass** der Vektor einen starken eukaryontischen Promotor zur Expression der insertierten Sequenz umfasst.

5. Rekombinanter Lebendimpfstoff nach Anspruch 4, **dadurch gekennzeichnet, dass** der starke Promotor ein Immediate-Early-CMV-Promotor, bevorzugt der murine oder humane Immediate-Early-CMV-Promotor, ist.

6. Rekombinanter Lebendimpfstoff nach einem der Ansprüche 1 bis 5, **dadurch gekennzeichnet, dass** er mindestens zwei Nucleotidsequenzen umfasst, die unter der Kontrolle verschiedener eukaryontischer Promotoren in die ORF5- oder ORF2-Region insertiert wurden.

7. Rekombinanter Lebendimpfstoff nach Anspruch 6, **dadurch gekennzeichnet, dass** die eukaryontischen Promotoren Immediate-Early-CMV-Promotoren verschiedenen tierischen Ursprungs sind.

8. Rekombinanter Lebendimpfstoff nach Anspruch 6, **dadurch gekennzeichnet, dass** er eine erste mit dem Immediate-Early-CMV-Promotor assoziierte Nucleotidsequenz und eine weitere mit einem anderen Promotor assoziierte Nucleotidsequenz umfasst, wobei die 5'-Enden der beiden Promotoren aneinandergrenzen.

9. Rekombinanter Lebendimpfstoff nach einem der Ansprüche 1 bis 8, **dadurch gekennzeichnet, dass** er eine ein antigenes Polypeptid codierende Nucleotidsequenz umfasst, wobei die Sequenz in die ORF5- und/oder ORF2-Region insertiert ist.

10. Rekombinanter Lebendimpfstoff nach Anspruch 9, **dadurch gekennzeichnet, dass** er eine Nucleotidsequenz umfasst, die ein antigenes Polypeptid eines Krankheitserregers der Katze codiert, wobei die Sequenz in die ORF5- und/oder ORF2-Region insertiert ist.

11. Rekombinanter Lebendimpfstoff nach Anspruch 10, **dadurch gekennzeichnet, dass** er eine Sequenz umfasst, die ein Antigen codiert, das ausgewählt ist aus der Gruppe der Antigene des felinen infektiösen Peritonitisvirus, des Katzenleukämievirus, des felinen Immunodeficiency-Virus, des felinen Panleukopenievirus und des felinen Calicivirus, wobei die Sequenz in die ORF5- und/oder ORF2-Region insertiert ist.

12. Rekombinanter Lebendimpfstoff nach Anspruch 10, **dadurch gekennzeichnet, dass** er eine Nucleotidsequenz umfasst, die aus den Nucleotidsequenzen ausgewählt ist, die das Polypeptid M, das modifizierte Polypeptid S oder das Polypeptid N von FIPV codieren, wobei die Sequenz in die ORF5- und/oder ORF2-Region insertiert ist.

13. Rekombinanter Lebendimpfstoff nach Anspruch 10, **dadurch gekennzeichnet, dass** er mindestens eine Nucleotidsequenz umfasst, die ausgewählt ist aus der Gruppe der Sequenzen, die den Antigenen env, gag, pol des felinen Leukämievirus, den Antigenen env, gag, pol des felinen Immunodeficiency-Virus, dem VP2-Kapsidantigen des felinen Panleukopenievirus, dem Kapsidantigen des felinen Calicivirus, den Antigenen M, modifiziertem S, 7b, der Polymerase des felinen infektiösen Peritonitisvirus entsprechen.

14. Rekombinanter Lebendimpfstoff nach den Ansprüchen 8 und 12 in ihrer Gesamtheit, **dadurch gekennzeichnet, dass** die unter der Kontrolle des Immediate-Early-CMV-Promotors insertierte Nucleotidsequenz eine Nucleotidsequenz ist, die das Polypeptid M, N oder das modifizierte Polypeptid S des FIPV-Virus codiert, und **dadurch**, dass die unter der Kontrolle des assoziierten Promotors insertierte Nucleotidsequenz eine Nucleotidsequenz ist, die ein Antigen einer anderen Katzenkrankheit codiert.

15. Rekombinanter Lebendimpfstoff nach einem der Ansprüche 1 bis 14, **dadurch gekennzeichnet, dass** er eine Nucleotidsequenz umfasst, die einen immunmodulatorisches Polypeptid codiert, wobei die Sequenz in die ORF5- und/oder ORF2-Region insertiert ist.

16. Rekombinanter Lebendimpfstoff nach Anspruch 15, **dadurch gekennzeichnet, dass** die Nucleotidsequenz aus der Gruppe der Cytokin-codierenden Sequenzen ausgewählt ist.

17. Multivalente Impfstoffformulierung, die mindestens zwei - als Gemisch vorliegende oder zu mischende - rekombinante Lebendimpfstoffe, wie in einem der Ansprüche 1 bis 16 definiert, umfasst, wobei die Impfstoffe verschiedene insertierte Sequenzen umfassen.

18. DNA-Fragment, das mindestens eine Sequenz umfasst, ausgewählt aus der Gruppe bestehend aus:
- der durch die Positionen 1 bis 8193 von SEQ ID NO:1 definierten Sequenz;
- der durch die Positionen 1655 bis 2596 von SEQ ID NO:1 definierten Sequenz der ORF2-Region;
- der durch die Positionen 5869 bis 7113 von SEQ ID NO:1 definierten Sequenz der ORF5-Region;
- den flankierenden Sequenzen von 100 bis 800 bp, die stromaufwärts und stromabwärts der ORF2-Region in SEQ ID NO:1 liegen;
- den flankierenden Sequenzen von 100 bis 800 bp, die stromaufwärts und stromabwärts der ORF5-Region in SEQ ID NO:1 liegen.

19. Verwendung eines Impfstoffs nach einem der Ansprüche 1 bis 16 oder einer Impfstoffformulierung nach Anspruch 17 zur Herstellung eines Impfstoffs für Katzen, der zur parenteralen oder oralen Verabreichung bestimmt ist.

20. Verwendung nach Anspruch 19, wobei der Impfstoff für Katzen zur oronasalen Verabreichung bestimmt ist.

21. Verwendung nach Anspruch 19 oder 20, wobei die Impfstoffdosis 10² DICC50 bis 10⁷ DICC50 ist.

22. Verwendung nach einem der Ansprüche 19 bis 21, wobei der Impfstoff für Katzen zur einmaligen Verabreichung bestimmt ist.
